# EUROPEAN PATENT APPLICATION

(11) **EP 3 614 393 A1**
(43) Date of publication of application: **26.02.2020**
(21) Application number: 19192375.4
(22) Date of filing: 19.08.2019
(51) Int. Cl.: G16H 40/63, G16H 50/20, G16H 50/70, G16H 40/67

(54) **REMOTE BIOMETRIC MONITORING AND COMMUNICATION SYSTEM**

(30) Priority: 20.08.2018 US 201862764990 P; 31.12.2018 US 201862786836 P; 15.08.2019 US 201916541800
(71) Applicant: Biosense Webster (Israel) Ltd., Yokneam, 2066717 (IL)
(72) Inventor: Govari, Assaf, Yorkneam 2066717 (IL); Fuchs, Amit, Yorkneam 2066717 (IL); Altmann, Andres Claudio, Yorkneam 2066717 (IL)
(74) Representative: Small, Gary James

(57) **Abstract**

Methods, apparatus, and systems for medical procedures are disclosed herein and include an external or implantable monitoring and processing apparatus that includes a memory, a sensor and a processor. The processor may be configured to receive biometric patient data from the sensor and transmit, via a first network, the biometric patient data based on a triggering cause. A local computing device may be configured to receive the biometric patient data via the first network, generate a combined patient data based at least on the received biometric patient data, and transmit the combined patient data via a second network. A remote computing system configured to receive the combined patient data via the second network, determine that a critical event is detected based on the combined patient data, and initiate a critical event action based on determining that the critical event is detected.

## Description

### FIELD OF INVENTION

The present application provides systems, apparatuses, and methods for improving medical procedures, diagnosis and treatment.

### CROSS REFERENCE TO RELATED APPLICATION

This application claims the benefit of U.S. provisional application No.62/764,990 filed on August 20, 2018, and U.S. provisional application No. 62/786,836 filed on December 31, 2018which are incorporated by reference as if fully set forth.

### BACKGROUND

Advances in wired and wireless communication have facilitated improvements in the transfer of data across various distances and have helped streamline the use and analysis of such data. The medical field relies on patient monitoring, diagnosis, and treatment and benefits from more efficient and robust means of conducting the same.

### SUMMARY

The present disclosure provides systems, apparatuses and methods for remotely monitoring and communicating various patient biometrics for use with different medical modalities to monitor, diagnose, and treat various disease states. The present disclosure provides systems, apparatuses and methods for that include an external or implantable monitoring and processing apparatus that includes a memory, a sensor and a processor. The processor may be configured to receive biometric patient data from the sensor and transmit, via a first network, the biometric patient data based on a triggering cause. A local computing device may be configured to receive the biometric patient data via the first network, generate a combined patient data based at least on the received biometric patient data, and transmit the combined patient data via a second network. A remote computing system configured to receive the combined patient data via the second network, determine that a critical event is detected based on the combined patient data, and initiate a critical event action based on determining that the critical event is detected. The memory may be configured to store the biometric data for at least one of a pre-determined time or a dynamically determined time. The combined patient data may also be based on at least one of an additional data or the triggering cause. The monitoring and processing apparatus may be configured to be in a general operation mode or a continuous operation mode and may include a transceiver configured to transmit biometric patient data at a first frequency based on the general operation mode or a second frequency based on the continuous operation mode.

An additional sensing apparatus may be provided and may be configured to receive a request for an additional data from the local computing device, sense the additional data and transmit, via the first network, the additional data.

A critical event action may include transmitting, via the second network, a critical event action instruction including contacting a healthcare professional, setting the monitoring and processing apparatus to a continuous operation mode, or providing instructions to a user. The critical event action instruction may be received, via the second network and at the local computing device, and an action may be performed based on the critical event action instruction.

Biometric patient data sensed by a sensor may be received at a local computing device and via a first network, based on a triggering cause. A combined patient data may be generated based at least on the received biometric patient data and the combined patient data may be transmitted via a second network. A critical event action may be determined based on the combined patient data and received from a remote computing device, at the local computing device and via the second network. The first network may be a local area network and the second network may be a wide area network. The combined patient data may further be based on at least one of an additional data or the triggering cause. The sensor may be part of a monitoring and processing apparatus. The critical event action may include contacting a healthcare professional, setting a monitoring and processing apparatus to a continuous operation mode, or providing instructions to a user.

A monitoring and processing apparatus is disclosed. The apparatus may include a patch including an adhesive component, an electronic device comprising electronic circuitry, a first portion configured to receive a removable electronic device and a second portion coupled to the first portion and separated from the first portion by a gap, the second portion including an electrode extending from the first portion to the second portion across the gap. The electronic device may be electronically coupled to the attachable patch via the at least one electrode. The electronic device may be electrically connected to the electrode by at least one conductive via disposed between the electronic circuitry and the electrode. The electronic device may be configured to receive at least one of a user input and a biometric patient data and may include a transceiver configured to transmit the biometric patient data. The transceiver may be configured to transmit the biometric patient data based on the user input.

### BRIEF DESCRIPTION OF THE DRAWINGS

A more detailed understanding can be had from the following description, given by way of example in conjunction with the accompanying drawings wherein:
FIG. 1 is a block diagram of an example system for remotely monitoring and communicating patient biometrics;
FIG. 2 is a block diagram of an example monitoring and processing apparatus, external to the patient;
FIG. 3A is a partial exploded view of an example monitoring and processing apparatus, including a patch and a removable electrical device;
FIG. 3B is a top view of the patch shown in FIG. 3A;
FIG. 3C is an illustration of an example system including a plurality of the monitoring and processing apparatuses shown in FIGs. 3A and 3B in wireless communication with each other;
FIG. 3D is a close-up view of the monitoring and processing apparatuses shown in FIG. 3C wired to a ground electrode;
FIG. 3E is a top view of an example of the removable electrical device shown in FIG. 3A-3D;
FIG. 4 is a block diagram of an example of the local computing device shown in FIG. 1;
FIG. 5A is an illustration of a computer-generated electrical signal and information associated with the monitored electrical signal displayed at an example local computing device with a display;
FIG. 5B is an illustration of the computer-generated electrical signal and information associated with the monitored electrical signal displayed at the local computing device shown in FIG. 1 including an icon embedded in the signal to show synchronization with the monitoring and processing apparatus;
FIG. 5C is an illustration of an electrical signal and information associated with the monitored electrical signal displayed at the computing device shown in FIG. 1.
FIG. 5D is an illustration of an example local computing device including displayed alerts of a detected event;
FIG. 6 is a system diagram of an example of a computing environment, which includes the computing system shown in FIG. 1;
FIG. 7 is a flow diagram of an example method of remotely monitoring and communicating patient biometrics;
FIG. 8 is a diagram of an example system for remotely monitoring and communicating patient biometrics;
FIG. 9 is a diagram of a database that stores various data and rules;
FIG. 10 is a flow diagram of an example method of event detection;
FIG. 11A is an illustration an example message displayed at a local computing device upon the detection of a user input;
FIG. 11B is an illustration of an example message displayed at a local computing device upon the detection of a sensed anomaly in the physiology of a patient user;
FIG. 11C is an illustration of an example message displayed at a local computing device upon the detection of an anomaly in the monitoring and processing apparatus;
FIG. 12A is an illustration of an example display displaying a non-critical event message at the local computing device;
FIG. 12B is an illustration of an example display displaying an example of a message prompting an input of additional information;
FIG. 12C is an illustration of an example message sent to a healthcare professional;
FIG. 13A is an illustration of an example message sent to a user when a critical event is detected;
FIG. 13B is an illustration of an example display of real-time data from the sensors;
FIG. 13C is an illustration of an example display of patient information about the user to first responders;
FIG. 14A is diagram of an example of a health care provider dashboard system;
FIG. 14B is an illustration of an example of a health care provider dashboard;
FIG. 14C is an illustration of an example of a patient details page;
FIG. 14D is an illustration of an example of an event details page;
FIG. 15A is a diagram of an example of a patient dashboard system;
FIG 15B is an illustration of an example of a user interface that is accessible to the user via the patient portal;
FIG. 15C is an illustration of an example of a record detail page;
FIG. 15D is an illustration of another example of a record detail page;
FIG. 15E is an illustration of another example of a record detail page;
FIG. 15F is an illustration of another example of a record detail page; and
FIG. 15G is an illustration of an example of an event detail page.

### DETAILED DESCRIPTION

Conventional systems and methods for monitoring and recording patient biometrics include sensing devices that record patient biometrics over a period of time (e.g., 24 hours or more). The recorded biometrics for the entire period are reviewed by a health professional (e.g., a physician). Some conventional systems include communication devices that provide (e.g., via a network) alerts regarding abnormal episodes that are detected by sensors (e.g., electrodes) of the sensing devices.

In some situations, a patient may feel a sensation in their body (e.g., an abnormal or uncomfortable feeling) that is not detected by the sensors. In these situations, the felt activities are not automatically provided by the sensing device to health professionals at the time of the feeling. The patients themselves must either immediately contact the health professional, record the time of the sensation or remember the time of the sensation to provide the time of the sensation to a health professional at a later time, which is burdensome to the patients. In addition, some felt sensations may indicate immediate or impending serious health issues (e.g., heart attack, stroke). Failure to promptly provide a health professional of the felt but not detected activities may result in an increased risk of serious injury or death to the patients.

Embodiments described herein provide systems, apparatuses, and methods for remotely monitoring various biometric patient activity (e.g., electrocardiogram (ECG) signals, electroencephalography (EEG) signals, Electromyography (EMG) signals, blood pressure, temperature and other measurable biometrics) for use with different medical modalities (e.g., ECG monitoring, pressure monitoring, orthopedics, and pain management treatment) for treatment across various disease states.

Embodiments described herein include one or more monitoring and processing apparatuses that continuously or periodically monitor the patient biometrics (i.e., patient data) and temporarily store (e.g., in buffer memory) the patient data for a period of time (e.g., 1 hour). Upon detection of an event (e.g., a user input, a detected arrhythmia, a loss of contact or connection), the one or more monitoring and processing apparatuses may store a portion of the patient data at and within a range of time before and after the detected event to non-volatile memory. Additionally, the one or more monitoring and processing apparatus may wirelessly communicate, via a short-range network (e.g., local area network (LAN) or personal area network (PAN)), the portion of the patient data to an electronic local computing device in synchronous communication with the monitoring and processing apparatus. The electronic local computing device may display all or a portion of the patient data and information associated with the patient data (e.g. diagnosis information, additional information from another device, etc.), and communicate, via a long-range network (e.g., wide area network (WAN), the internet, a cellular network), at least some of the patient data and the associated information to a remote computing system monitored by a healthcare professional (e.g., a physician).

The system disclosed herein may analyze data and, according to thresholds and parameters dynamically calibrated by the system or physician-determined thresholds and parameters, may provide the physician and/or patient with alerts, additional information, and/or instructions. The system disclosed herein may enable and facilitate communication in various forms (e.g., portal alerts, pages, short range communication, long range communication, emails, texts alerts, and social media messaging) between the physician and the patient. The system may provide the physician with a dashboard of patient statuses, such that the physician may concentrate on those patients having more critical needs than others.

Some conventional systems include devices, external to the patient, (e.g., patches adaptable to be positioned on the surface of a patient) that sense patient data, such as ECG signals. These devices may include multiple leads (e.g., 12-leads), which may cause the devices to be bulky and cumbersome to the patient. For example, in an environment such as a gym or a health club, these bulky multi-lead patches may be inconvenient to the patient during physical activity. Embodiments described herein provide a system that includes a plurality of leadless monitoring and processing apparatuses, including patches in wireless communication with each other. In one embodiment, at least one of the platforms mounted on the patches has an extensible lead to a third electrode, and this third electrode may be used as a ground for the 12-lead system. In some embodiments, the extensible lead and its attached electrode may be retained within the platform on a spring-loaded wheel.

Some conventional patches are activated upon sensing a tapping of the patch using an accelerometer. However, accelerometers are costly and consume significant power, and their relatively large sizes may limit the sizes and number of other elements of the patch.

Embodiments described herein provide efficient (e.g., less costly, more power efficient, and/or smaller) monitoring and processing apparatuses that sense a user input via a capacitive sensor. The smaller sized capacitive sensors may be more easily integrated with other components of the apparatus and may facilitate a smaller apparatus profile. Further, in some examples, capacitive sensors are configured to respond selectively to different tapping patterns, (e.g., a single tap or a double tap), facilitating a more robust apparatus because different tasks of the patch, such as acquisition, storing, or transmission of data, can be activated in response to the different tapping patterns.

Embodiments described herein provide a monitoring and processing apparatus that includes an attachable patch (e.g., attachable to the skin of a patient), having one or more electrodes for acquiring electrical signals from the patient, and a removable electronic device (i.e., removably coupled to the patch) having circuitry that is electrically connected to the one or more electrodes through one or more conductive vias when coupled to the patch. The patch may include at least one portion having an electrode that is printed onto a flexible substrate, forming the at least one portion of the patch. The patch may also include a second portion, separated from the first portion by a gap, having a more rigid substrate than the substrate of the at least one first portion. The second portion may be configured to receive the removable electronic device. While the electronic device is coupled to the first portion of the patch, the electronic processing device may be electrically connected to the one or more electrodes through the one or more conducted vias.

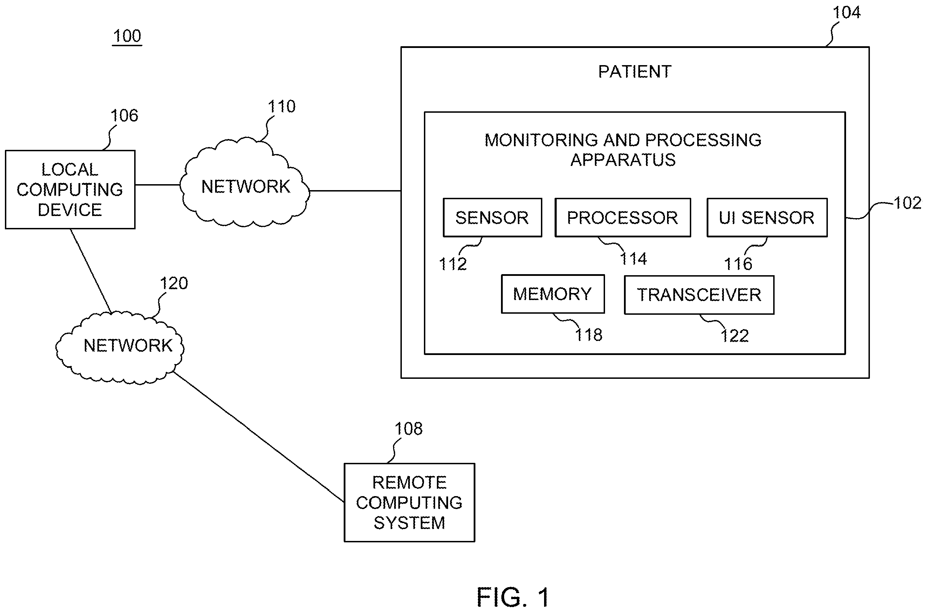

FIG. 1 is a block diagram of an example system 100 for remotely monitoring and communicating patient biometrics (i.e., patient data). In the example illustrated in FIG. 1, the system 100 includes a patient biometric monitoring and processing apparatus 102 associated with a patient 104, a local computing device 106, a remote computing system 108, a first network 110 and a second network 120.

According to an embodiment, a monitoring and processing apparatus 102 may be an apparatus that is internal to the patient's body (e.g., subcutaneously implantable). The monitoring and processing apparatus 102 may be inserted into a patient via any applicable manner including orally injecting, surgical insertion via a vein or artery, an endoscopic procedure, or a laparoscopic procedure.

According to an embodiment, a monitoring and processing apparatus 102 may be an apparatus that is external to the patient. For example, as described in more detail below, the monitoring and processing apparatus 102 may include an attachable patch (e.g., that attaches to a patient's skin). The monitoring and processing apparatus 102 may also include a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, a monitoring and processing apparatus 102 may include both components that are internal to the patient and components that are external to the patient.

A single monitoring and processing apparatus 102 is shown in FIG. 1. Example systems may, however, may include a plurality of patient biometric monitoring and processing apparatuses. A patient biometric monitoring and processing apparatus may be in communication with one or more other patient biometric monitoring and processing apparatuses. Additionally or alternatively, a patient biometric monitoring and processing apparatus may be in communication with the network 110.

One or more monitoring and processing apparatuses 102 may acquire patient biometric data (e.g., electrical signals, blood pressure, temperature, blood glucose level or other biometric data) and receive at least a portion of the patient biometric data representing the acquired patient biometrics and additional formation associated with acquired patient biometrics from one or more other monitoring and processing apparatuses 102. The additional information may be, for example, diagnosis information and/or additional information obtained from an additional device such as a wearable device. Each monitoring and processing apparatus 102 may process data, including its own acquired patient biometrics as well as data received from one or more other monitoring and processing apparatuses 102.

In FIG. 1, network 110 is an example of a short-range network (e.g., local area network (LAN), or personal area network (PAN)). Information may be sent, via short-range network 110, between monitoring an processing apparatus 102 and local computing device 106 using any one of various short-range wireless communication protocols, such as Bluetooth, Wi-Fi, Zigbee, Z-Wave, near field communications (NFC), ultraband, Zigbee, or infrared (IR).

Network 120 may be a wired network, a wireless network or include one or more wired and wireless networks. For example, a network 120 may be a long-range network (e.g., wide area network (WAN), the internet, or a cellular network,). Information may be sent, via network 120 using any one of various long-range wireless communication protocols (e.g., TCP/IP, HTTP, 3G, 4G/LTE, or 5G/New Radio).

The patient monitoring and processing apparatus 102 may include a patient biometric sensor 112, a processor 114, a user input (UI) sensor 116, a memory 118, and a transmitter-receiver (i.e., transceiver) 122. The patient monitoring and processing apparatus 102 may continually or periodically monitor, store, process and communicate, via network 110, any number of various patient biometrics. Examples of patient biometrics include electrical signals (e.g., ECG signals and brain biometrics), blood pressure data, blood glucose data and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

Patient biometric sensor 112 may include, for example, one more sensors configured to sense a type of biometric patient biometrics. For example, patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer and a microphone).

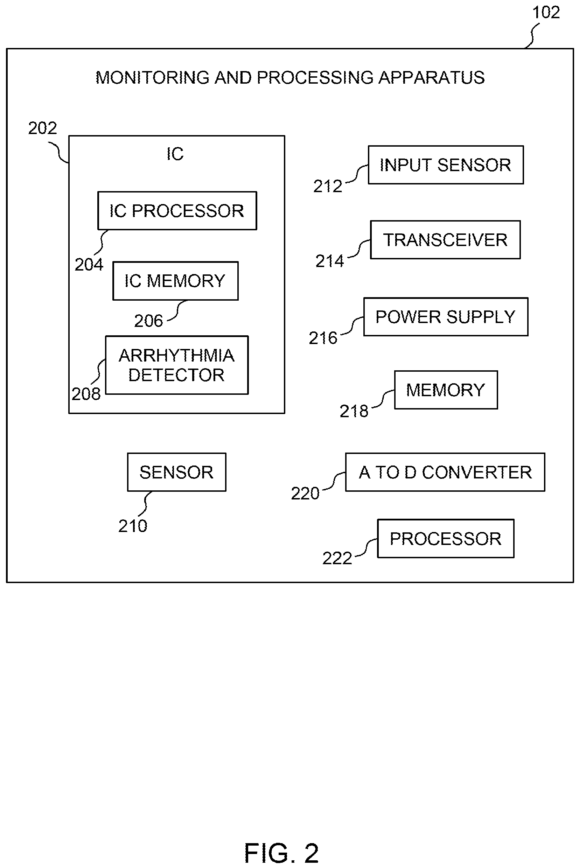

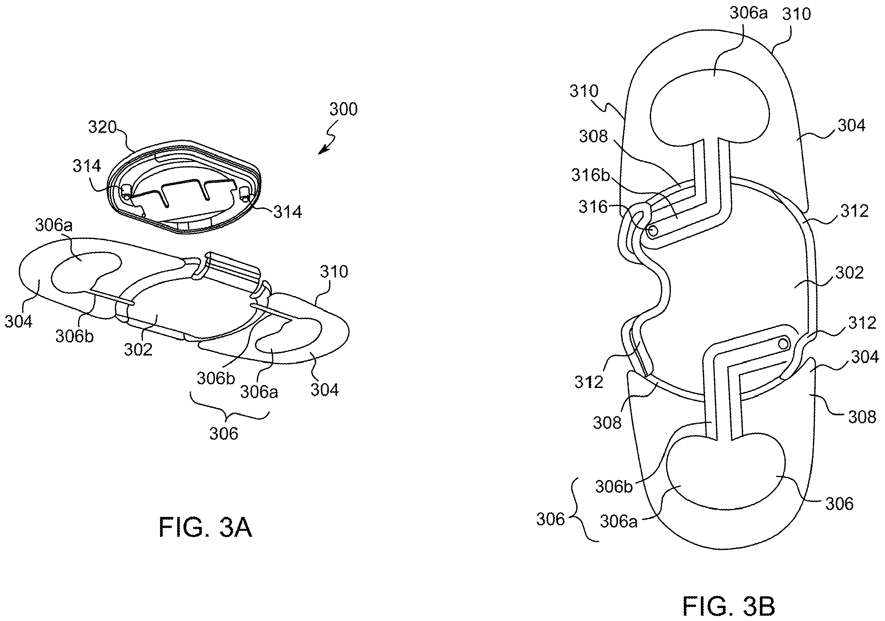

As described in more detail below with reference to FIGs. 2, 3A and 3B, patient biometric monitoring and processing apparatus 102 may be an ECG monitor for monitoring ECG signals of a heart. The patient biometric sensor 112 of the ECG monitor may include one or more electrodes for acquiring ECG signals. The ECG signals may be used for treatment of various cardiovascular diseases.

In another example, the patient biometric monitoring and processing apparatus 102 may be a continuous glucose monitor (CGM) for continuously monitoring blood glucose levels of a patient on a continual basis for treatment of various diseases, such as type I and type II diabetes. The CGM may include a subcutaneously disposed electrode, which may monitor blood glucose levels from interstitial fluid of the patient. The CGM may be, for example, a component of a closed-loop system in which the blood glucose data is sent to an insulin pump for calculated delivery of insulin without user intervention.

Transceiver 122 may include a separate transmitter and receiver. Alternatively, transceiver 122 may include a transmitter and receiver integrated into a single device.

Processor 114 may be configured to store patient data, such as patient biometric data in memory 118 acquired by patient biometric sensor 112, and communicate the patient data, across network 110, via a transmitter of transceiver 122. Data from one or more other monitoring and processing apparatus 102 may also be received by a receiver of transceiver 122, as described in more detail below.

According to an embodiment, the monitoring and processing apparatus 102 includes UI sensor 116 which may be, for example, a piezoelectric sensor or a capacitive sensor configured to receive a user input, such as a tapping or touching. For example UI sensor 116 may be controlled to implement a capacitive coupling, in response to tapping or touching a surface of the monitoring and processing apparatus 102 by the patient 104. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

As described in more detail below with regard to FIG. 2, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from processing apparatus 102 when a gesture is detected.

The local computing device 106 of system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via network. Alternatively, the local computing device 106 may be a stationary or standalone device, such as a stationary base station including, for example, modem and/or router capability, a desktop or laptop computer using an executable program to communicate information between the processing apparatus 102 and the remote computing system 108 via the PC's radio module, or a USB dongle. Patient biometrics may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, such as a local area network (LAN) (e.g., a personal area network (PAN)). In some embodiments, the local computing device 106 may also be configured to display the acquired patient electrical signals and information associated with the acquired patient electrical signals, as described in more detail below.

In some embodiments, remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, such as any of the wireless technologies mentioned above. As described in more detail below, the remote computing system 108 may be configured to provide (e.g., visually display and/or aurally provide) the at least one of the patient biometrics and the associated information to a healthcare professional (e.g., a physician).

FIG. 2 is a block diagram of a more detailed example of the monitoring and processing apparatus 102 of FIG. 1 used to monitor biometric user data, such as ECG signals of a heart. The sensor 210 may be an electrical signal sensor and may include one or more electrodes for acquiring signals, such as ECG signals, from a patient. According to an example, ECG signals collected by sensor 210 may be used for diagnostics of various cardiovascular diseases. The monitoring and processing apparatus 102 shown in FIG. 2 may monitor and process patient biometrics while external to the patient 104 (e.g., attached to the skin of the patient) or, alternatively, may monitor and process patient biometrics internal (e.g., subcutaneous) to the patient 104.

As shown in FIG. 2, the monitoring and processing apparatus 102 may include integrated circuit (IC) 202. The IC 202 may include IC processor 204, IC memory 206, and arrhythmia detector 208. The sensor 210, which may be an electrical signal sensor, may be a part of IC 202 as shown in FIG. 2 or may be external to IC 202 (not shown). The monitoring and processing apparatus 102 may also include off chip (i.e., separate from IC 202) components, which may include sensor 210, input sensor 212, power supply 216, memory 218 (e.g., off chip memory), analog to digital (A to D) converter 220, off-chip processor 222 and transceiver 214.

Sensor 210, which may be an off-chip component, may include one more sensors each configured to sense a type of patient biometric. For example, sensor 210 may include an electrode configured to acquire electrical biometrics (e.g., heart signals, brain signals, and/or other bioelectrical signals), a temperature sensor, a blood pressure sensor, blood glucose sensor, a blood oxygen sensor, a weight sensor, and/or sensors that acquire other medical and non-medical information, such as, for example, motion, elevation changes or acceleration.

Input sensor 212 may be, for example, a piezoelectric sensor or a capacitive sensor configured to receive a user input, such as a gesture, tapping, or touching. IC 202 may be configured, for example, to respond selectively to different tapping patterns of the UI sensor 116 of FIG. 1 or input sensor 212 of FIG. 2, (e.g., a single tap or a double tap) such that different tasks of the patch (e.g., acquisition, storing, or transmission of data) may be activated based on the detected pattern. For example, processor 222, which may be an off-chip component, may be configured to decode the pattern of taps in the signal from input sensor 212, which may be a capacitive sensor, and to execute one of its processing functions depending upon the pattern. For example, one tap (single tap) may activate acquiring and storing the data from input sensor 212 into memory 218, two taps (double-tap) may activate transmitting the data stored in memory 218, and three taps (triple-tap) may activate both acquiring and storing the data into memory 218 and an immediate transmission of the same data by transceiver 214. Alternatively, other tapping patterns may be used to activate the data processing functions.

In some embodiments, the monitoring and processing apparatus includes a plurality of user input sensors 212. In these embodiments, the tapping patterns may be used to differentiate between data processing functions for the different user input sensors 212. Alternatively, off-chip processor 222 may respond to each of the taps identically, for example, by transmitting the patient biometrics via transceiver 214.

Transceiver 214 facilitates communication with the local computing device 106 using a short-range wireless communication standard (e.g., Bluetooth) and may be the same as transceiver 122 of FIG. 1. Transceiver 214 may include multiple transmitters and receivers to facilitate the short-range wireless communication. For example, transceiver 214 may include a transmitter and a receiver, configured to transfer information such as the monitored biometrics information from one or more other monitoring and processing apparatuses over a short-range wireless network such as network 110. Monitoring and processing apparatus 102 may also include separate transmitters and receivers. The transceiver 214 may be configured, for example, to modulate (at a particular frequency) information to be sent via an antenna (not shown).

Portions of memory 218 may include volatile and non-volatile memory, for example, random access memory (RAM), dynamic RAM, or a cache.

Referring to the IC 202 components, arrhythmia detector 208 may include hardware logic circuitry (e.g., logic gates, latches, and flip-flops) configured to detect an arrhythmia based on the monitored signals acquired by sensor 210, which may include one more sensors. IC processor 204 may include one or more processors configured to receive programmed instructions (software/code) and write parameters and configurations when triggered by an event (e.g., after arrhythmia is detected, receive user input, loss of connection or contact with the patient's body, upon request, or upon expiration of a time interval). IC memory 206 may include an amount of memory sufficient to facilitate an IC processor 204 to write parameters and configurations.

At least some function of processor 222 may be implemented upon the occurrence of an event. Accordingly, the amount of power consumed by processor 222, from the power supply 216, may be reduced and thereby the life of the monitoring and processing apparatus 102 may be prolonged. Power consumption may also be reduced because the hardware logic circuitry used to detect the arrhythmia and the processor's functions are limited to performing simple tasks of writing parameters and configurations. That is, because the processing apparatus 102 performs episode detection by itself, allowing to transmit and store a fraction of the monitored data that may have diagnostic significance, significant power may be saved when compared to conventional sensing devices, causing the life (e.g., battery life) of the processing apparatus 102 to be an order of magnitude greater than conventional sensing devices.

Processor 222, which may be an off-chip component, may be configured to record, in memory 218, the monitored patient biometrics received at transceiver 214. For example, monitored patient biometrics (e.g., electrical signals acquired via electrodes) may be continuously or periodically recorded, in real-time, to a buffer memory portion of memory 218. The stored patient biometrics may be maintained in the buffer memory for a predetermined or dynamically determined amount or range of time (e.g., 1 hour).

Processor 222 may also be configured to store detected events (e.g., arrhythmias detected by arrhythmia detector 208). In addition, upon the occurrence of a detected event, off-chip processor 222 may store the monitored electrical biometrics (e.g., temporarily stored in the buffer memory), occurring in a time range before and after the detected event, to a non-volatile memory portion of memory 218. Off-chip processor 222 may also be configured to provide alerts or indications of detected events (e.g., detected arrhythmia, loss of contact/electrical connection, low battery, or confirmation that user input (e.g., tap) is received). The alerts or indications may be provided to the patient by the local computing device 106 or sent to the remote computing system, via network 120, to be provided to a remove entity, such as a remote healthcare professional.

As described above with regard to FIGs. 1 and 2, according to an embodiment, monitoring and processing apparatuse 102 may monitor and process patient biometrics while external to the patient 104. FIGs. 3A and 3B illustrate an example monitoring and processing apparatus 300, which includes an attachable patch 310 and a removable electronic device 320. The monitoring and processing apparatus 300 may be the same as or similar to the monitoring and processing apparatus 102 of FIG. 1. FIG. 3A is a partial exploded view of an example monitoring and processing apparatus 300, which includes the attachable patch 310 and the removable electrical device 320. FIG. 3B is a top view of the attachable patch 310 shown in FIG. 3A.

Referring generally to FIGs. 3A and 3B, the attachable patch 310 includes a first portion 302 configured to receive the removable electronic device 320 and a pair of opposing second portions 304 adjacent to the first portion 302. The number of second portions 304 shown in FIG. 3A and 3B is merely an example. Attachable patches may include a single second portion as well as more than two second portions.

Each of the opposing second portions 304 may include an electrode 306 comprising an electrode pad 306a and an electrode pathway 306b extending from the pad 306a. The number of electrodes 306 shown at each second portion 304 in FIGs. 3A and 3B is merely an example. In other examples, second portions may also include a plurality of electrodes. The electrodes 306 may be configured to sense biometric data and transmit the biometric data to a removable electronic device 320, as described herein. As shown in FIG. 3B, each second portion 304 may be separated from the first portion 302 by a gap 308. Each electrode pathway 306b may extend from an electrode pad 306a across a gap 308, to the second portion 304, which is configured to receive the removable electronic device 320. The first portion 302 and the second portions 304 may be coupled together via coupling portions 312.

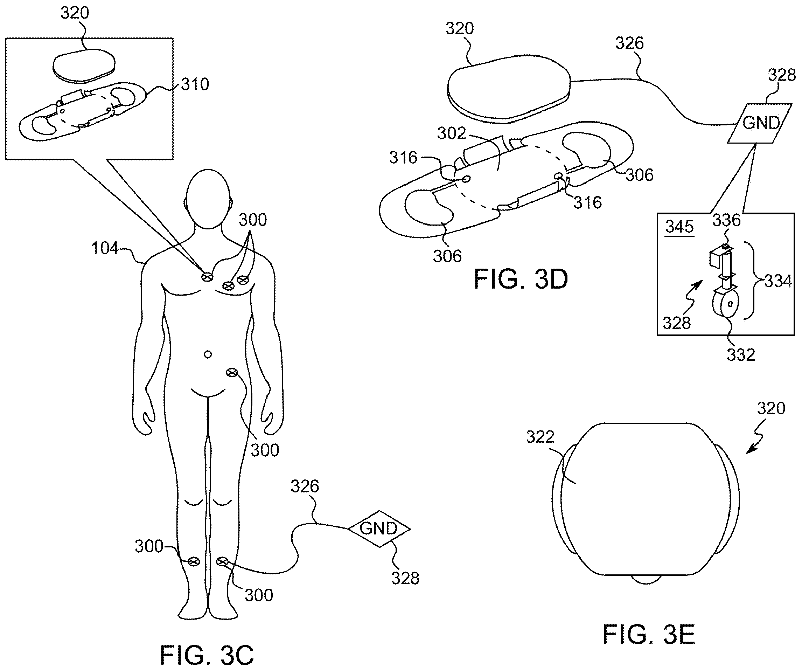

When the removable electronic device 320 is coupled to the first portion 302 of the patch 310, electronic components (e.g., one or more components described above with regard to FIG. 2) of the removable electronic device 320 may be electrically connected to the electrode pathways 306b at the first portion 302 through contacts 314 of the removable electronic device 320 and the conductive vias 316 (shown in FIGs. 3B and 3D). The removable electronic device 320 may be configured to receive monitored electric signals and/or user input (e.g., via capacitive sensing as described above with regard to FIG. 2), process the electrical signals and transmit the electrical signals upon at least one of a detection of the user input and a detection of an arrhythmia.

The removable electronic device 320 may include one or more components of monitoring and processing apparatus 102, as shown in FIG. 1. The one or more components may include a sensor 112, processor 114, UI sensor 116, memory 118, and transceiver 122.

The second portions 304 may include adhesive material to attach the second portions 304 to a patient. According to an embodiment, the first portion 302 may include a rigid substrate and the second portions 304 may include more flexible substrates, to facilitate maintaining contact with the patient and sensing of the electrical signals.

The size and location of the gaps 308 may be selected to limit movement of one portion (e.g., the first portion 302) caused by movement of another portion (e.g., a second portion 304) for maintaining physical and/or electrical contact with the patient. The gap 308 may also limit movement between the patch 310 and the removable electronic device 320.

FIG. 3C is a system diagram of an example of a plurality of the monitoring and processing apparatuses 300 shown in FIGs. 3A and 3B in wireless communication with each other. The embodiment shown in FIG. 3C may be, for example, used to monitor an occurrence of cardiac arrhythmia in a non-symptomatic subject. The embodiment shown in FIG. 3C may facilitate lighter and less cumbersome monitoring and processing apparatuses 300 for a patient, particularly during periods of more strenuous physical activity. According to an embodiment, the periods of more strenuous physical activity may be identified based on biometric data collected by sensor 210 of Fig. 2, which may indicate an accelerated heart rate, the patient 104's motion, or the like.

It will be understood that although cardiac related conditions are discussed herein, one or more monitoring and processing apparatuses 102 of FIGs. 1 and 2 may be implemented for monitoring and processing any patient related condition that can be detected based on the components of such monitoring and processing apparatuses 102, as described herein.

The monitoring and processing apparatuses 300 shown in FIG. 3C may use any one of various short-range wireless communication protocols (e.g., Bluetooth, Wi-Fi, Zigbee, Z-Wave, infrared (IR), near field communications (NFC), ultraband, Zigbee and infrared (IR)) to communicate with each other. Each of the monitoring and processing apparatuses 300 may also be in communication with a local computing device, such as the local computing device 106 described in FIG. 1.

One of the monitoring and processing apparatuses 300 shown in FIG. 3C may be wired to ground electrode 328 via extensible lead 326. FIG. 3D is a close-up view of the monitoring and processing apparatuses 300 wired to ground electrode 328. Inset 345 of FIG. 3D shows a schematic, pictorial illustration of ground electrode 328 implemented as a spring-loaded conductive wheel assembly 334, in accordance with an embodiment. Assembly 334 comprises a conductive wheel 332 configured to provide an electrical contact for grounding, such as to a moving surface of a treadmill (not shown).

A patch 310 may include adhesive material configured to allow the patch to be used as a one-time use or multi-time use patch. Each patch 310 may include, for example, printed electronics technology (e.g., the substrate upon which the electronics is printed includes a PET (polyethylene terephthalate) foil or paper glued on a fabric). Electrically conductive vias 316 may be processed through the substrate and fabric layers to electrically connect ECG electrodes that are printed onto the substrate of a patch to a metal connector of a respective detachable electronic device. The patch 310 may be attached, for example, to the skin.

The example of FIGs. 3A-3C illustrates monitoring and processing apparatuses external to patient 104. Systems may include multiple implantable (e.g., subcutaneous) and/or external monitoring and processing apparatuses 102 that may be in communication with each other. Each monitor and processing apparatus 102 may process data, including its own acquired patient biometrics and data received from one or more other apparatuses.

The embodiment shown in FIG. 3C provides superior signal quality while facilitating comfortable sensing and communication devices for use in a non-medical environment and, therefore, may increase the probability of early detection of medical conditions such as cardiac abnormalities in, for example, otherwise non-symptomatic subjects that are experiencing cardiac arrhythmia during physical activity.

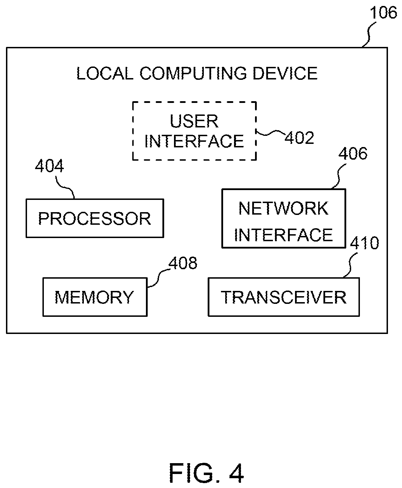

FIG. 4 is a block diagram of an example of the local computing device 106 shown in FIG. 1. As described above, the local computing device 106 may be a mobile device (e.g., smart phone, smart watch, tablet or other portable smart device configured to communicate with other devices via network). Alternatively, the local computing device 106 may be a stationary device (e.g., a stationary base station including for example, modem and/or router capability, a desktop or laptop computer or other dedicated standalone device). As shown in FIG. 4, the local computing device 106 may include one or more of a user interface 402, a processor 404, a network interface 406 (e.g., for a mobile device or a stationary device with a network connection), memory 408, and a transceiver 410. The network interface 406, memory 408, and transceiver 410 can be configured to perform the same tasks as those described above in FIG. 2. Accordingly, the description of these components is omitted with regard to FIG. 4.

User interface 402 may be, for example, a touch screen configured to display information, such as patient biometrics, and/or receive user inputs. Processor 404 may be configured to control the user interface to execute an application that displays the monitored patient biometrics received from monitoring and processing apparatus 102. Alternatively or additionally, processor 404 may also be configured to provide an indication to a sensor 210 of FIG. 2. The indication may be a signal (e.g., activation or wake up signal, activity signal, or inactivity signal), data, or the like.

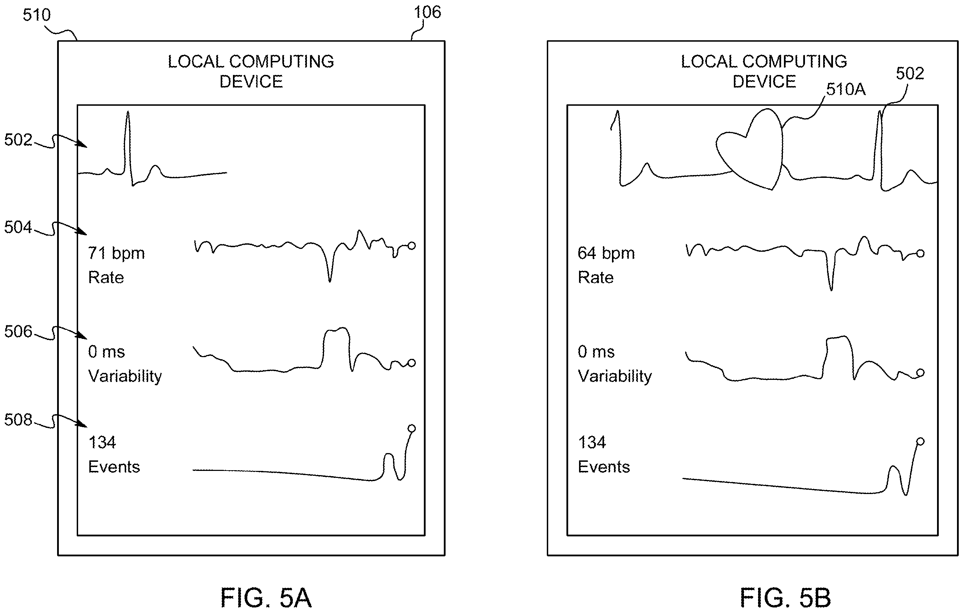

FIGs. 5A and 5B show an example of providing an indication that a local computing device 106 may be synchronized with the monitoring and processing apparatus 102. FIG. 5A is an illustration of an example local computing device 106 having a display 520 including a displayed computer-generated signal 502 and display portions 504, 506 and 508 for displaying different types of information. FIG. 5B is an illustration of the example local computing device 106 showing the displayed computer-generated signal 502 with an embedded heart icon 510A. The displayed computer-generated signal 502 shown in FIG. 5B is at a point in time after the computer-generated signal 502 is displayed at FIG. 5A. The embedded heart icon 510A may indicate to the user that the local computing device 106 is synchronized with the monitoring and processing apparatus 102. The displayed information may also include other visualizations to indicate a status to the user, such as whether the recently acquired biometric data are healthy/normal or abnormal. When the local computing device 106 is synchronized with the monitoring and processing apparatus 102, communication between local computing device 106 and the monitoring and processing apparatus 102 is established. The synchronization may include handshaking between the two devices to establish protocols (e.g., NFC) of a communication link at the start of the communication.

In one example, the local computing device 106 is a mobile device and is initialized (e.g., synchronized with the removable electrical device 320 of FIG. 3) by bringing the local computing device 106 and the removable electrical device 320 close to each other to establish protocols (e.g., NFC) of the communication link. In another example, a Quick Response (QR) code may be disposed on the removable electrical device 320 and on the local computing device 106. For example, the QR code may be disposed in addition to or alternative to the image 322 on the removable electrical device 320 shown in FIG. 3E. In one example, the QR code may be embedded into an image (e.g., image 322). The local computing device 106 may be initialized by using the local computing device to read the QR code (e.g., via an application on the local computing device 106).

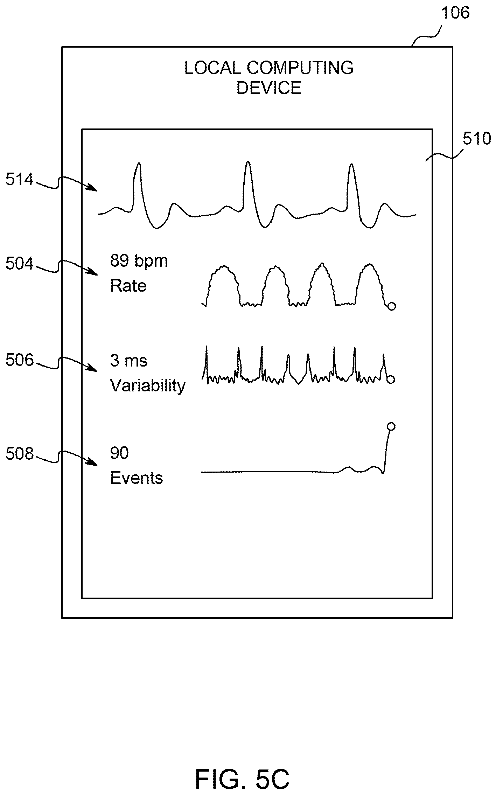

FIG. 5C is an illustration of an example local computing device 106 having a display 510 including a monitored electrical signal 514 and information associated with the monitored electrical biometrics. For example, rate information (e.g., beats per minute (bpm)) is displayed at a rate information portion 504 of the display 510, variability information (in milliseconds) displayed at a variability information portion 506 of the display 510 and event information (e.g., number of detected arrhythmias over a period of time) is displayed at an event information portion 508 of the display 510. This information is shown in a numerical format as well as a graphical format. It will be noted that the information associated with patient heart biometrics shown in FIG. 5A - 5C is merely example information and the data, the fields, and the characteristics may be different based on patient, application, and programing. Other types of information associated with patient biometrics may also be displayed. Local computing device 106 may display information related to any biometric data across any number of various disease states.

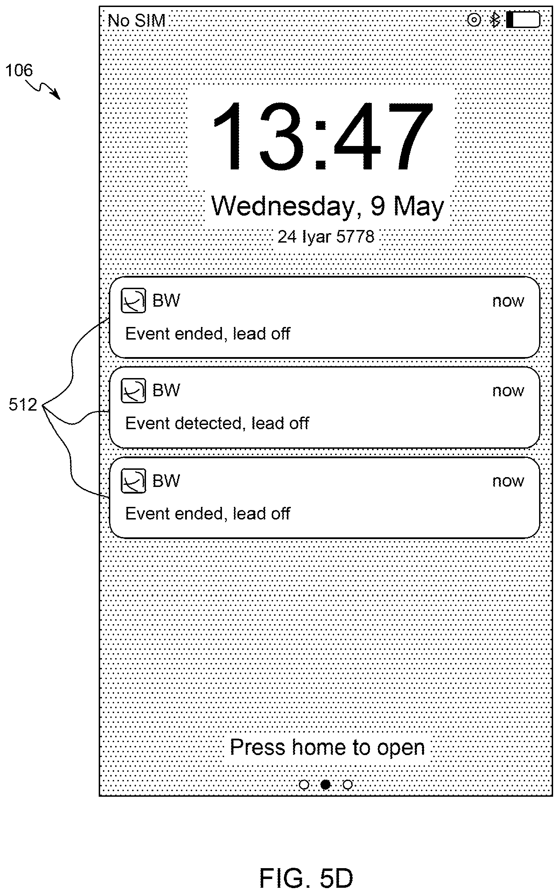

According to an implementation, alerts, updates, or changes related to a patient (e.g., patient biometric data) may be provided via local computing device 106. FIG. 5D is an illustration of an example local computing device 106 including example displayed alerts of a detected event. For example, as shown in FIG. 5D, alerts 512 may be displayed indicating the detection of an event. As shown in FIG. 5D, the event is a lead being disconnected. Three separate alerts are displayed, indicating the ending of a first event, the detection of a second event and the ending of second event.

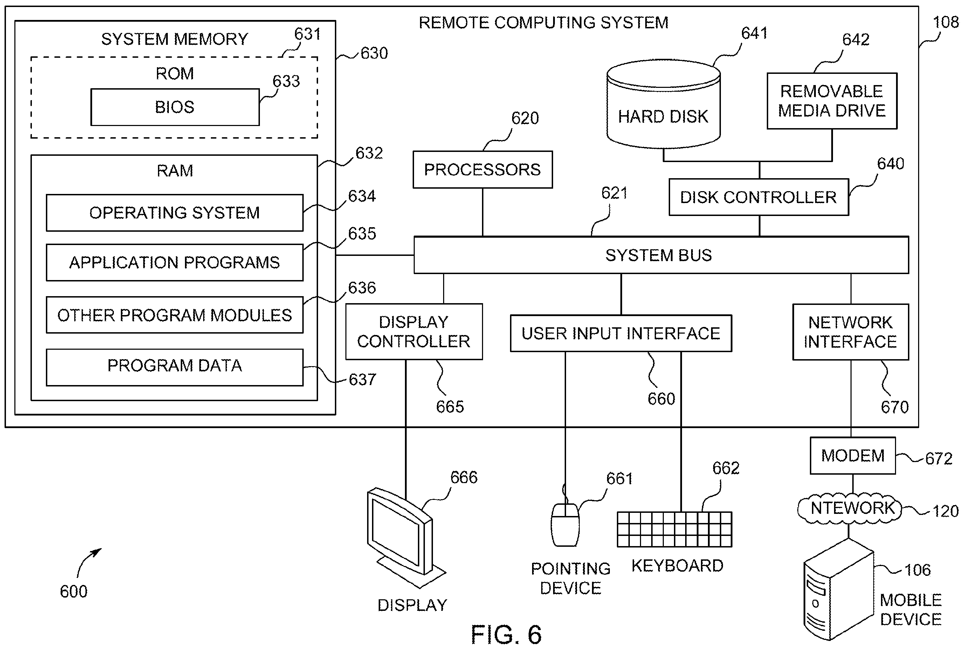

FIG. 6 is a system diagram of an example of a computing environment 600 in communication with network 120. In some instances, the computing environment 600 is incorporated in a public cloud computing platform (such as Amazon Web Services or Microsoft Azure), a hybrid cloud computing platform (such as HP Enterprise OneSphere) or a private cloud computing platform.

As shown in FIG. 6, computing environment 600 includes remote computing system 108 (hereinafter computer system), which is one example of a computing system upon which embodiments described herein may be implemented.

The remote computing system 108 may, via processors 620, which may include one or more processors, perform various functions. The functions may include analyzing monitored patient biometrics and the associated information and, according to physician-determined or algorithm driven thresholds and parameters, providing (e.g., via display 666) alerts, additional information or instructions. As described in more detail below, the remote computing system 108 may be used to provide (e.g., via display 666) healthcare personnel (e.g., a physician) with a dashboard of patient information, such that such information may enable healthcare personnel to identify and prioritize patients having more critical needs than others.

As shown in FIG. 6, the computer system 610 may include a communication mechanism such as a bus 621 or other communication mechanism for communicating information within the computer system 610. The computer system 610 further includes one or more processors 620 coupled with the bus 621 for processing the information. The processors 620 may include one or more CPUs, GPUs, or any other processor known in the art.

The computer system 610 also includes a system memory 630 coupled to the bus 621 for storing information and instructions to be executed by processors 620. The system memory 630 may include computer readable storage media in the form of volatile and/or nonvolatile memory, such as read only system memory (ROM) 631 and/or random access memory (RAM) 632. The system memory RAM 632 may include other dynamic storage device(s) (e.g., dynamic RAM, static RAM, and synchronous DRAM). The system memory ROM 631 may include other static storage device(s) (e.g., programmable ROM, erasable PROM, and electrically erasable PROM). In addition, the system memory 630 may be used for storing temporary variables or other intermediate information during the execution of instructions by the processors 620. A basic input/output system 633 (BIOS) may contain routines to transfer information between elements within computer system 610, such as during start-up, that may be stored in system memory ROM 631. RAM 632 may contain data and/or program modules that are immediately accessible to and/or presently being operated on by the processors 620. System memory 630 may additionally include, for example, operating system 634, application programs 635, other program modules 636 and program data 637.

The illustrated computer system 610 also includes a disk controller 640 coupled to the bus 621 to control one or more storage devices for storing information and instructions, such as a magnetic hard disk 641 and a removable media drive 642 (e.g., floppy disk drive, compact disc drive, tape drive, and/or solid state drive). The storage devices may be added to the computer system 610 using an appropriate device interface (e.g., a small computer system interface (SCSI), integrated device electronics (IDE), Universal Serial Bus (USB), or FireWire).

The computer system 610 may also include a display controller 665 coupled to the bus 621 to control a monitor or display 666, such as a cathode ray tube (CRT) or liquid crystal display (LCD), for displaying information to a computer user. The illustrated computer system 610 includes a user input interface 660 and one or more input devices, such as a keyboard 662 and a pointing device 661, for interacting with a computer user and providing information to the processor 620. The pointing device 661, for example, may be a mouse, a trackball, or a pointing stick for communicating direction information and command selections to the processor 620 and for controlling cursor movement on the display 666. The display 666 may provide a touch screen interface that may allow input to supplement or replace the communication of direction information and command selections by the pointing device 661 and/or keyboard 662.

The computer system 610 may perform a portion or each of the functions and methods described herein in response to the processors 620 executing one or more sequences of one or more instructions contained in a memory, such as the system memory 630. Such instructions may be read into the system memory 630 from another computer readable medium, such as a hard disk 641 or a removable media drive 642. The hard disk 641 may contain one or more data stores and data files used by embodiments described herein. Data store contents and data files may be encrypted to improve security. The processors 620 may also be employed in a multi-processing arrangement to execute the one or more sequences of instructions contained in system memory 630. In alternative embodiments, hard-wired circuitry may be used in place of or in combination with software instructions. Thus, embodiments are not limited to any specific combination of hardware circuitry and software.

As stated above, the computer system 610 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 620 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 641 or removable media drive 642. Non-limiting examples of volatile media include dynamic memory, such as system memory 630. Non-limiting examples of transmission media include coaxial cables, copper wire, and fiber optics, including the wires that make up the bus 621. Transmission media may also take the form of acoustic or light waves, such as those generated during radio wave and infrared data communications.

The computing environment 600 may further include the computer system 610 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 610. When used in a networking environment, computer system 610 may include modem 672 for establishing communications over a network 120, such as the Internet. Modem 672 may be connected to system bus 621 via network interface 670, or via another appropriate mechanism.

Network 120, as shown in FIGs. 1 and 6, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 610 and other computers (e.g., local computing device 106).

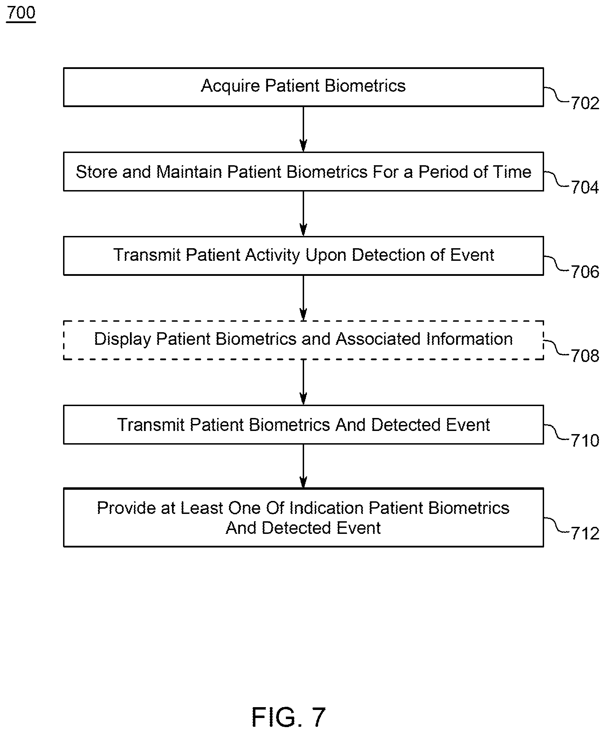

FIG. 7 is a flow diagram of a method of remotely monitoring and communicating patient biometrics. As shown at block 702 in FIG. 7, the method 700 includes continuously, intermittently or periodically acquiring patient biometric data of a patient at a patient biometric monitoring and processing apparatus such as monitoring and processing apparatus 102 of FIG. 1 and monitoring and processing apparatus 300 of FIGs. 3A-3D.

As shown at block 704 in FIG. 7, the method 700 includes storing the patient biometrics and maintaining the patient biometrics in memory for a period of time. The patient biometrics may be stored at memory 118 of monitoring and processing apparatus 102, as shown in Fig. 1. The patient biometrics stored in memory, at block 704, may include raw data collected by, for example, patient biometric sensor 112 or may be processed data processed by processor 114.

As shown at block 706 in FIG. 7, the method 700 includes transmitting (e.g., via a LAN), upon detection of an event, all or a portion of the patient biometric data stored and maintained at block 704. The all or portion of the patient biometric data that is transmitted may be patient biometric data that is collected or that occurs within a time range before and/or after the detection of the event. As described herein, the event may be a medical condition as determined by processor 114 of monitoring and processing apparatus 102 of FIG. 1, the occurrence of a threshold biometric data event, an event identified as such by local computing device 106, or the like. The patient biometric data stored and maintained at block 704 and transmitted at block 706 may be transmitted wirelessly or using a wire connection via network 110 of FIG. 1.

As shown at block 708 in FIG. 7, the method 700 includes displaying, at a local computing device 106, the portion of the patient biometric data and/or information associated with the portion of the patient biometric data. For example, a local computing device may receive patient biometric data and execute, or make available for execution, an application that displays the portion of the patient biometric data and/or information associated with the portion of the biometric patient biometrics.

As shown at block 710 in FIG. 7, the method 700 includes transmitting, via a large area network, the detected event and the portion of the biometric patient biometrics. The transmission of block 710 may be facilitated via local computing device 106 and network 120 of FIG. 1.

As shown at block 712 in FIG. 7, the method 700 includes providing, at a remote computing system, at least an indication of the detected event, all or a the portion of the biometric patient biometrics, or information related to the detected event. The remote computing system may be same as or similar to the remote computing system as described in FIGs. 1 and 6. As an example, the detected event and the portion of the patient biometric data may be received, at the remote computing system that is remote from the patient biometric monitoring and processing apparatus (e.g., monitoring and processing apparatus 102 of FIG.1) and the local computing device (e.g., local computing device 106 of FIG. 1). The portion of the biometric patient data may be displayed at the remote computing system. In addition, an alert may be provided to the remote computing system in the form of a text, email, social media message, sound, vibration, notification, or the like.

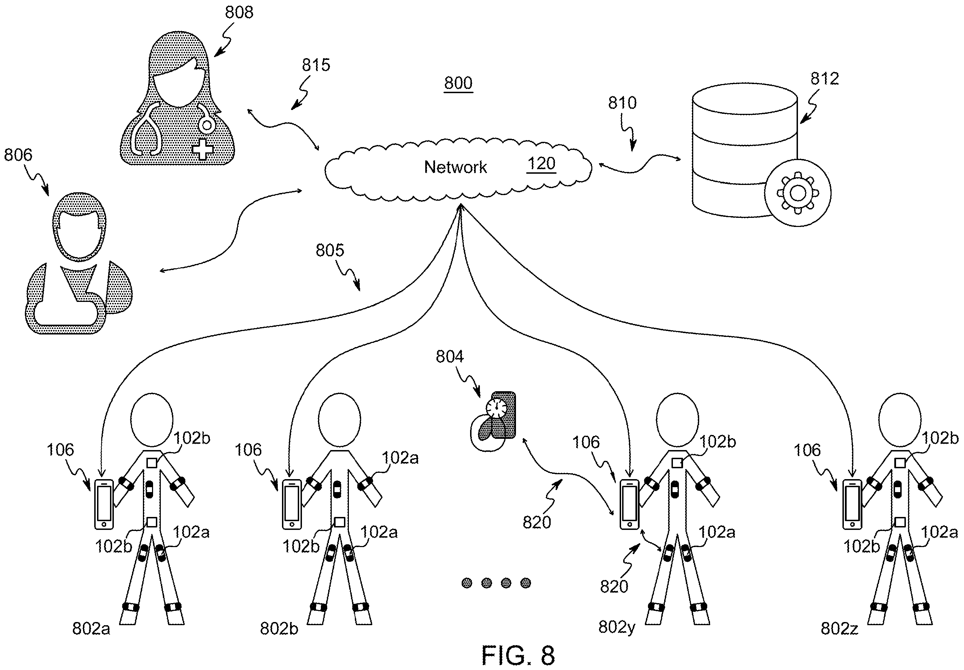

FIG. 8 is a diagram of an example system 800. As shown in the example system 800 in FIG. 8, each local computing device 106 of the system 800 is a mobile device. As described above, local computing devices 106 may be a mobile device or a stationary device. For simplified explanatory purposes, each local computing device 106 in FIG. 8 is described as a mobile device. Each local computing device 106 in FIG. 8 is in communication with attachable monitoring and processing apparatuses 102a and implantable monitoring and processing apparatuses 102b associated with one of the users 802a ... 802z. For example, information may be sent between each of the local computing devices 106 and associated external monitoring and processing apparatuses 102a and 102b via wireless communication channels 820 using any one of various short-range wireless communication protocols. Wireless communication channels 820 may be the same as or similar to the network 110 of FIG. 1.

As shown in FIG. 8, a plurality of attachable monitoring and processing apparatuses 102a (e.g., monitoring and processing apparatus 102 shown in FIG. 1 or monitoring and processing apparatus 300 shown in FIGs. 3A-3D) and/or a plurality of implantable monitoring and processing apparatuses 102b (e.g., implanted subcutaneously within a patient) may be used to monitor patient biometric data of each user 802. The number of attachable monitoring and processing apparatuses 102a and implantable monitoring and processing apparatuses 102b shown in FIG. 8 is merely an example. Any number of attachable monitoring and processing apparatuses 102a and implantable monitoring and processing apparatuses 102b may be used. The location of the attachable monitoring and processing apparatuses 102a and implantable monitoring and processing apparatuses 102b shown in FIG. 8 are also example locations. Attachable and implantable monitoring and processing apparatuses may be disposed in locations different from those shown in FIG. 8.

In addition to or alternative to the monitoring and processing apparatuses 102a and monitoring and processing apparatuses 102b shown in FIG 8, an external sensing apparatus 804 may be optionally used to monitor patient biometric data and may communicate with an associated local computing device 106 via one of the wireless communication channels 820. Examples of external sensing apparatuses 804 include a blood pressure cuff, a weight scale, a wearable such as a fitness tracker or smart watch biometrics trackers, a glucose monitor, a CPAP machine or virtually any device which may provide an input concerning the health or activity of the patient. Although a single external sensing apparatus 804 is shown in FIG. 8, examples include any number of external sensing apparatuses 804 in communication with an associated mobile device. Further, although FIG. 8 shows a local computing device 106 that is a single mobile device in communication with external sensing apparatus 804, examples include one or more external sensing apparatuses 804 in communication with any number of associated mobile devices.

Each local computing device 106 is connected to network 120 (e.g., the Internet) via a wireless communication channel 805. Network 120 is also shown in FIG. 1. As shown in FIG. 8, the system 800 also includes a doctor portal 808 and a patient portal 806 connected to the network 120 via one or more communication channels 815. A cloud database 812, which may be part of the remote computing system 108, is also connected to the network 120 via communication channel 810. Each of the doctor portal 806, the patient portal 808 and the cloud database 812 may be implemented using one or more of the components of the computing environment 600 shown in FIG. 6. In some instances, the cloud database 812 is implemented by a public cloud computing platform (such as Amazon Web Services, or Microsoft Azure), a hybrid cloud computing platform (such as HP Enterprise OneSphere) or private cloud computing platform.

Information may be sent across each of the wireless communication channels 805, 810 and 815 using various short-range wireless communication protocols (e.g., Wi-Fi), various long-range wireless communication protocols (e.g., TCP/IP, HTTP, 3G,, 4G (LTE), 5G (New Radio)) or a combination of various short-range and long-range wireless communication protocols. Information may be sent using any one of various modes, such as e-mail, texting, voice over IP (VoIP) and conventional telephone communication.

The doctor portal 808 may allow a doctor or other medical services provider to access information that is stored in the cloud database 812. Similarly, the patient portal 806 may allow the patient to access information that is stored in the cloud database 812. In some instances, both the doctor portal 808 and the patient portal 806 are accessed via a standard computer browser. In other instances, both the doctor portal 808 and the patient portal 806 are accessed via a proprietary application that runs on either a computer or a mobile device. The doctor portal 808 and the patient portal 806 may be provided by any applicable web server.

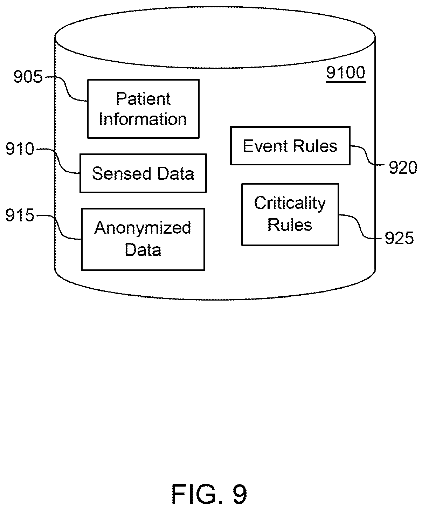

The cloud database 812 may include a database (shown in Fig. 9) that includes a plurality of tables. Examples of the plurality of tables that may be stored in the database 9100 are illustrated in FIG. 9. In some instances, the cloud database 812 includes a machine learning system such as a neural network. The machine learning system uses the information stored in the database 9100 to identify correlations between sensor data stored in the database 9100 and medical events.

FIG. 9 illustrates a database 9100 that stores various data and rules used to implement features of the disclosure. For example, database 9100 may include a patient information table 905 comprising biographical information about each user 802. This information may include, for example, name, date of birth, allergies, current medications, and emergency contact information. The information stored in the patient information table 905 may be stored in compliance with General Data Protection Regulation (GDPR) or Health Insurance Portability and Accountability Act (HIPAA requirements) regulations.

Database 9100 may further include a sensor data table 910 that stores data measured by the monitoring and processing apparatuses 102a and 102b and any external sensing apparatuses 804. The data stored in 910 is associated with the patient information stored in 905. The data in anonymized data table 915 includes the same data as 910 but is anonymized to remove any identifiable information that could be used to identify a particular user 802.

The database 9100 further stores a table of event rules 920. The event rules stored in the table of event rules 920 may define correlations and associations between sensor data and the occurrence of medical events. For example, an event rule may define that the user 802 is experiencing a blot clot when a blood pressure on a left arm of the user 802 is measuring a different pressure than a blood pressure sensor on the right arm of the user 802. Another example rule may define that the user 802 is hypoglycemic when a glucose sensor measures a low value and a heart rate sensor measures an increase in heart rate. A further example rule may define that the user 802 is having a stroke when the sensor data indicates both a high heart rate and high blood pressure.

The event rules may be predefined based on known medical correlations. Alternatively, the plurality of rules may be dynamically determined by the cloud database 812 using machine learning or other artificial intelligence techniques. For example, the cloud database 812 may use the anonymized data table 915 to identify correlations between the sensor data and the medical conditions.

The plurality of event rules stored in table 920 may also include rules that define that a sensor is malfunctioning. For example, if two EKG sensors were to measure different patterns, this may suggest that at least one of the sensors is malfunctioning.

The database 9100 may also store a table 925 of criticality rules. The criticality rules may determine if a particular medical event is critical and requires immediate medical attention. Examples of rules stored in table 925 include rules that define a minor heart arrhythmia, detected in a patient known to suffer from atrial fibrillation, to be a non-critical event, and rules that define the same minor heart arrhythmia as a critical event when it occurs in an otherwise healthy patient. In some examples, the rules defining the criticality of the event may be predetermined based on known medical relationships. Alternatively, the criticality of the event may be dynamically determined by the cloud database 812 using machine learning algorithms run on the anonymized data stored in table 915.

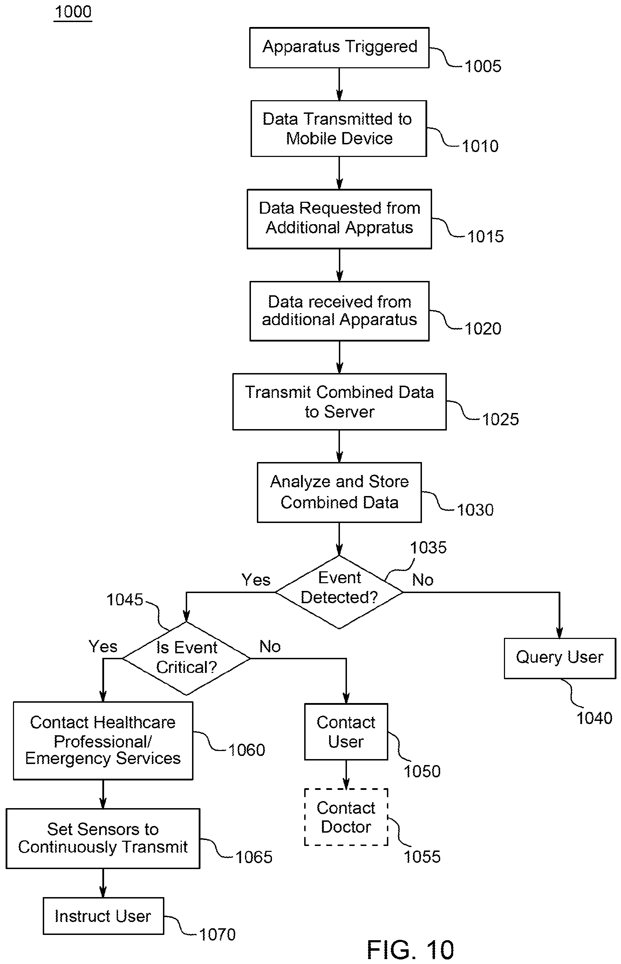

FIG. 10 is a flow diagram of a method 1000. As shown at block 1005 of method 1000, a monitoring and processing apparatus 102 is triggered based on a triggering cause. For example, the triggering cause may be a user input (e.g., touching or tapping by the user 802 of FIG. 8) on the apparatus 102. Alternatively, the triggering cause may be when the monitoring and processing apparatus 102 detects an anomaly in the physiology of the user 802. For example, in the case where the monitoring and processing apparatus 102 monitors blood glucose levels, an anomaly may be detected with the glucose levels of the user 802 are above or below critical levels. A triggering cause may be in response to the occurrence of an event, or a schedule, or in accordance with other criteria.

In examples where the monitoring and processing apparatus 102 is a blood pressure sensor, the triggering cause may be an anomaly detected when the blood pressure of the user 802 blood drops below or rises above critical levels. In some examples, the triggering cause may be an anomaly of the monitoring and processing apparatus 102. For example, the monitoring and processing apparatus 102 may detect an anomaly when a lead of the sensor becomes disconnected from the user 802 or the charge of the power supply of the monitoring and processing apparatus 102 is below a power threshold.

As shown at block 1010, the monitoring and processing apparatus 102 may transmit the data stored in the memory 118 to the local computing device 106. The data stored in the memory 118 of the monitoring and processing apparatus 102 may include measurements taken of the user 802 during a predefined time period (e.g., 5 minutes, 1 hour, 12 hours or 24 hours). Alternatively, the memory 118 may store all of the data since the monitoring and processing apparatus 102 last transmitted data to the local computing device 106. The monitoring and processing apparatus 102 may use Bluetooth, ZigBee or any other wireless communication standard to transmit the data to the local computing device 106.

In response to receiving the data from the monitoring and processing apparatus 102, the local computing device 106 may request data from one more additional sensing apparatuses (e.g., 102a, 102b and 804 of FIG. 8), as shown at block 1015. An additional sensing apparatus may be, for example, of the same type as the monitoring and processing apparatus 102. Alternatively, the additional sensing apparatus may be of a different type than monitoring and processing apparatus 102. In yet other instances, the additional sensing apparatus may be configured to monitor both the same biometric patient data as monitoring and processing apparatus 102 and different biometric patient data than the monitoring and processing apparatus 102. For example, the additional sensing apparatuses may be configured to monitor EKG, glucose, blood pressure, glucose, lactic acid, weight, activity (i.e., number of steps), oxygenation, blood flow, temperature or any other type of biometric patient data that measures the biological condition of the user 802.

As shown in block 1020, the data may be received by the local computing device 106 from the additional sensing apparatuses (e.g., 102a, 102b and 804). The data received from the additional sensing apparatuses may include blood pressure, heart rate, glucose levels, lactic acid levels, temperature, oxygenation or other measurements of the physical condition of the user 802. In block 1025, the local computing device may combine the data from the additional sensing apparatus 804 with the data from the monitoring and processing apparatus 102 to form combined data. The local computing device 106 may then transmit the combined data to the cloud database 812. The local computing device may use any wireless communication technology known in the art (such as Wi-Fi, 4G, 5G, etc.) to transmit the combined data via the internet. According to an embodiment, block 1015 and 1020 may be omitted such that data from an additional apparatus is not requested or received. According to this embodiment, at block 1025, the data that is transmitted to the server is the data or is based on the data from block 1010. According to an embodiment, data may be requested from an additional apparatus at block 1015 but no such data may be provided and, thus, received at block 1020. According to this embodiment, at block 1025, the data that is transmitted to the server is the data or is based on the data from block 1010.

The combined data may then be received by the cloud database 812 of FIG. 8. The cloud database 812 may then store the combined data in the database 9100 at block 1030. In some instances, the cloud database 812 may store an additional copy of the data that has been anonymized to form anonymized data stored at the anatomized data table 915 of FIG.9. The anonymized data may be formed to comply with the General Data Protection Regulation (GDPR) or Health Insurance Portability and Accountability Act (HIPAA requirements) regulations. The anonymized data may be used by the cloud database as training data for machine learning algorithms.

The cloud database 812 of FIG. 8 may then analyze the combined data received from the local computing device 106 by applying the plurality of event rules stored in 920 of FIG. 9.

Based on the results of the analysis in block 1030, the cloud database 812 may determine whether an alarming event has occurred in block 1035. Examples of alarming events include the detection of a period of exertion by the user 802 (e.g., during a workout), detection of lead of the monitoring and processing apparatus 102 becoming disconnected, detection that a negative health event has occurred in the user 802 such as a stroke, heart attack, arrhythmia, or atrial fibrillation.

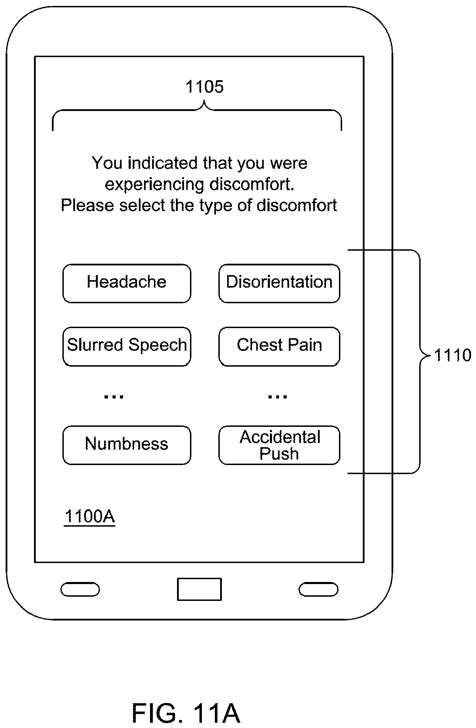

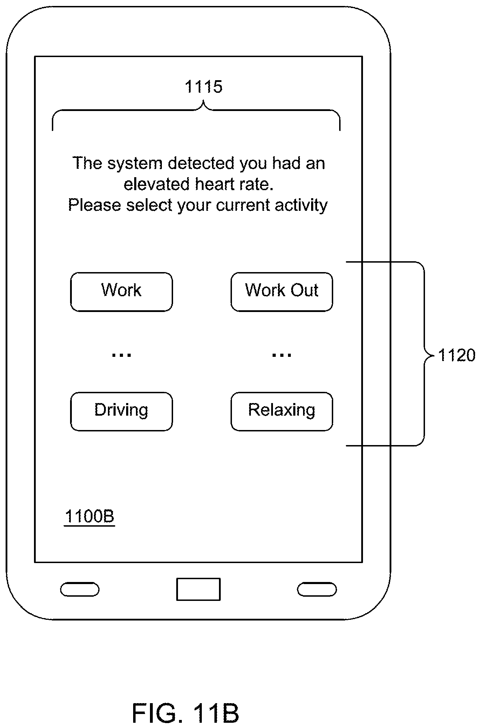

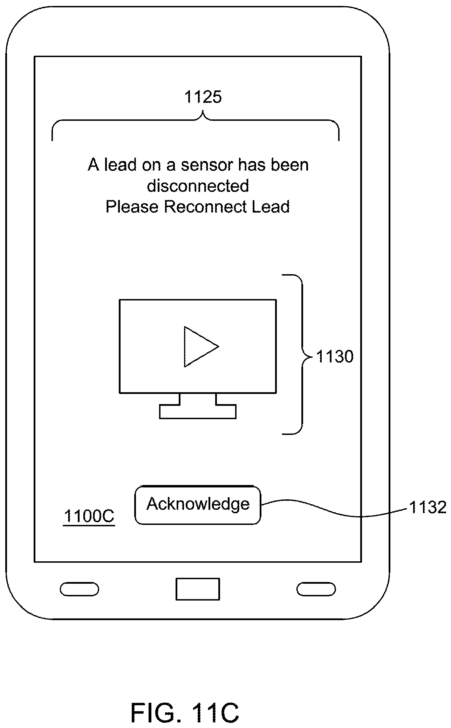

In some instances, if an alarming event is not detected by the cloud database 812 at block 1035, the user 802 may be queried by sending a message to the local computing device 106, as shown at block 1040. The contents of the message sent to the local computing device 106 may be based on the type of event that triggered block 1005. FIGs. 11A, 11B and 11C are displays 1100A, 1100B and 1100C showing example contents displayed at a local computing device 106. The user query, as shown at block 1040, may be sent if a threshold for an alarming event being detected, at 1035, is not met but if a secondary threshold that indicates a potential event is met such that a query may enable confirmation of the event. Further, a user query, as shown at block 1040, may be provided if the apparatus is triggered, at 1005, by a user action (e.g., tap or gesture). It should be noted that a query, as shown at block 1040, may not always be sent to a user based on an event not being detected at 1035.

For example, message 1105 in FIG. 11A may be displayed at the local computing device 106 when the alarming event is a button press. FIG. 11A shows an example of display 1100A that may be displayed on the local computing device 106 when the cloud database 812 does not detect an alarming event and the triggering cause is a user input (e.g., via user input sensor 212). The display 1100A may display the message 1105 from the cloud database 812 to the user 802. The display 1100A may also include a plurality of selectable answers 1110, each providing a reason for the user input. For example, an answer may indicate that the user accidentally tapped the removable electronic device 320. The local computing device 106 may record the answer selected by the user 802 and transmit the selection to the cloud database 812 for further storage and analysis.

As another example, if the triggering cause is a sensed anomaly in the physiology of the user 802, an example message shown in FIG. 11B may be displayed by the local computing device 106. When the triggering cause is an anomaly in the sensor, an example message shown in FIG. 11C may be displayed by the local computing device 106.

FIG. 11B shows an example of a display 1100B may be displayed on the local computing device 106 when the detected anomaly is an elevated heart rate. In this example, the message 1115 alerts the user 802 to the occurrence of the triggering cause and prompts the user 802 for additional information. The user 802 may be able to enter the additional information by selecting one of a plurality of options 1120. For example, the options may allow the user to select that the cause of elevated heart rate was from the stress of driving or from a gym workout. The local computing device 106 may record the selection of a particular option by the user 802 and transmit the selection to the cloud database 812 for further storage and analysis.

FIG. 11C shows an example of a display 1100C that may be displayed on the local computing device 106 when the triggering cause is an anomaly in the monitoring and processing apparatus 102. In the example illustrated in FIG. 11C, an example display is shown for when the anomaly is a disconnected lead. In this example, the message 1125 alerts the user 802 to the occurrence of the event and prompts an acknowledgement from the user via acknowledge selector 1132. In some instances, a video tutorial 1130 is displayed to the user 802 to instruct the user on how to connect the leads. When the leads are reconnected, an acknowledgement can be sent via the acknowledge selector 1132.

The cloud database 812 may send the message at block 1040 to the local computing device 106 using any of variety communication protocols and communication modes. The message may be received at the local computing device 106 and displayed at the local computing device 106 via, for example, an integrated or external display. The message may prompt the user 802 to select an answer to one or more additional questions or provide additional information using the local computing device 106. This additional information may then be transmitted back to the cloud database for further storage and analysis.

If an event is detected at decision block 1035, the cloud database 812 may determine the criticality of the detected event based on the criticality rules stored in 925. If the event is determined, at block 1045, by the cloud database 812 to be non-critical, the user may be contacted, as shown at block 1050. The user 802 may be contacted by sending a message to the local computing device 106. According to an embodiment, one or more factors such as user preferences, event type, or prior history, may be applied when determining whether the user is contacted at 1050 if the event detected at 1035 is determined not to be a critical event at 1045.

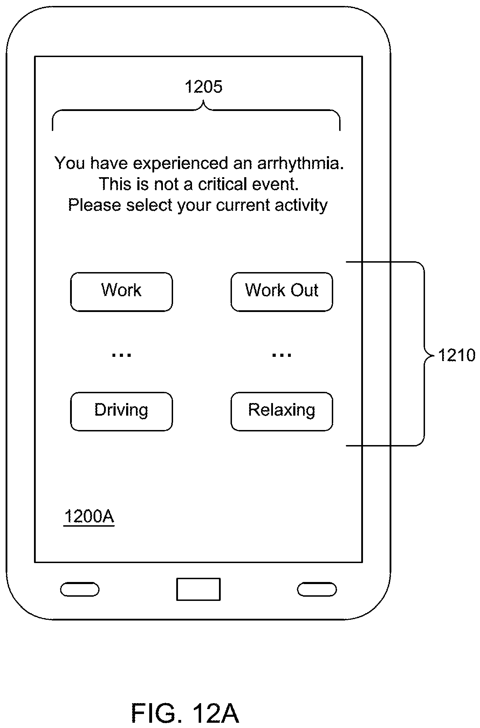

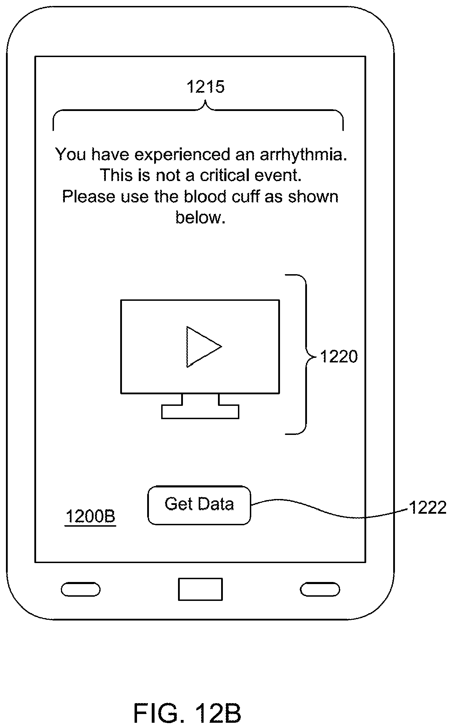

In some instances, the message that is sent to the user device in block 1050 may request additional information about the event from the user 802. For instance, FIG. 12A shows an example where the user 802 enters additional information by selecting prompts. In other instances, such as shown in FIG. 12B, the additional information is entered using an external sensor 804. This additional information may then then be transmitted back to the cloud database 812 for further storage and analysis.

In the example display 1200A, the user 802 is alerted, via message 1205, of a detected arrhythmia. The user 802 is also prompted to select additional information describing the activity of the user 802 when then event occurred using selectable answers 1210. The local computing device 106 may record the selection of a particular option by the user 802 and transmit the selection to the cloud database 812 for further storage and analysis.

In the example illustrated in FIG. 12A, an example of contents displayed if the cloud database 812 determines additional data (e.g., from an external sensing apparatus 804) to be input from an external sensing apparatus 804 is provided. For example, FIG. 12B shows a display 1200B that is displayed when additional data is to be input from a blood pressure cuff. The message 1215 informs the user 802 that the arrhythmia has occurred. In addition, the message 1215 instructs the user to provide additional data via the blood cuff. In some instances, a video 1220 is displayed to the user 802 instructing the user 802 on the proper use of the external sensing apparatus 804. When the user 802 uses the external sensing apparatus 804 and an input is received via Get Data selector 1222, the local computing device 106 records the data from the external sensing apparatus 804 and transmits the data to the local computing device 106 where it can be further transmitted to the cloud database 812 for further storage and analysis via the local computing device 106.

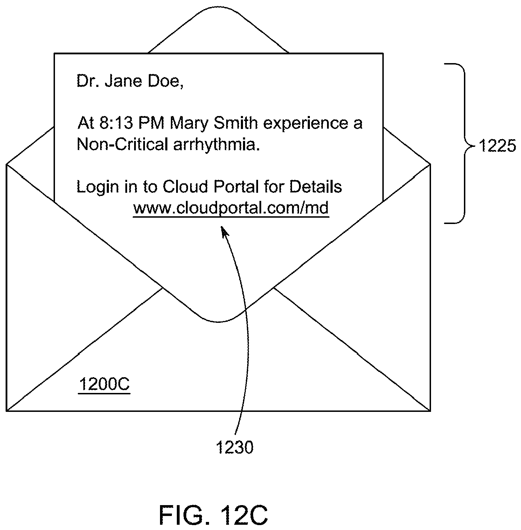

According to an embodiment, if a non-critical event is detected in 1045, a healthcare professional (e.g., a doctor) may be contacted, at block 1055. The healthcare professional may be contacted via a short message service, email message, a platform, or the like. The contact may include information 1225 about the non-critical event as a well as a link 1230 for the healthcare professional to log in to the doctor portal 806 of the cloud database 812. An example message 1200C sent to a doctor is shown in FIG. 12C. According to an embodiment, one or more factors such as user preferences (e.g., doctor, healthcare facility, and/or platform preference), event type, prior history, or the like may be applied when determining whether the healthcare professional is contacted at 1055, if the event detected at 1035 is determined not to be a critical event at 1045.

FIG. 12C illustrates an example message 1200C that may be sent to the doctor in block 1250. The message 1200C may be in the form of an email or SMS message. The example message 1200C includes information 1225 describing the non-critical event. In addition, the example message 1200C includes a link 1230 to the doctor portal 806 of the cloud database 812. In some instances, the message may include a plot of the time series data that triggered the non-critical event.

If the event is determined, at block 1045, to be critical (e.g., by the cloud database 812), a critical event action may be initiated. A critical event action may be transmitting an instruction (e.g., by the cloud database 812) that results in contacting of a healthcare professional at block 1060, setting one or more sensors to continuously transmit 1065, providing instruction to a user at block 1070, or the like, as further described below.

A healthcare professional and/or an emergency service may be contacted at block 1060. For example, an emergency service provider may be contacted by causing the local computing device 106 to send an SMS to the emergency services provider via a Text to 911 system. Additionally or alternatively, for example, the emergency service provider may be contacted by causing the local computing device 106 to dial the emergency services provider (e.g., dial 911 in the United States) and play a message describing the critical event to the emergency services provider.

As shown at block 1060, the healthcare professional may also be contacted if the event is determined to be critical, at 1045. For example, a doctor may be contacted via an email or text message. Additionally or alternatively, the cloud database 812 may place a phone call to the healthcare professional and play a message describing the critical event to the healthcare professional. By having the cloud database 812 call the doctor, the local computing device 106 is free to contact the emergency services provider.

As shown in block 1065, the cloud database may also instruct the local computing device 106 to set the monitoring and processing apparatus 102 and the additional sensing apparatuses 804 to continuously output their measurements. These measurements may be received by the local computing device 106 and streamed in real-time to the cloud database. According to an embodiment, the monitoring and processing apparatus 102 and/or additional sensing apparatuses 804 may be set to a first, general, operation mode prior to the cloud database instruction at step 1065. The monitoring and processing apparatus 102 and/or additional sensing apparatuses 804 may be set to a second, continuous, operation mode after the cloud database instruction at step 1065. The monitoring and processing apparatus 102 and/or additional sensing apparatuses 804 may remain in the second, continuous, operation mode for a predetermined amount of time or for an amount of time that is dynamically determined. The predetermined amount of time may be based on one or more factors such as user preferences (e.g., patient, doctor, healthcare facility, and/or platform preference), event type, prior history, or the like. The dynamically determined amount of time may be based on the sensor data and may be determined by the database 9100.

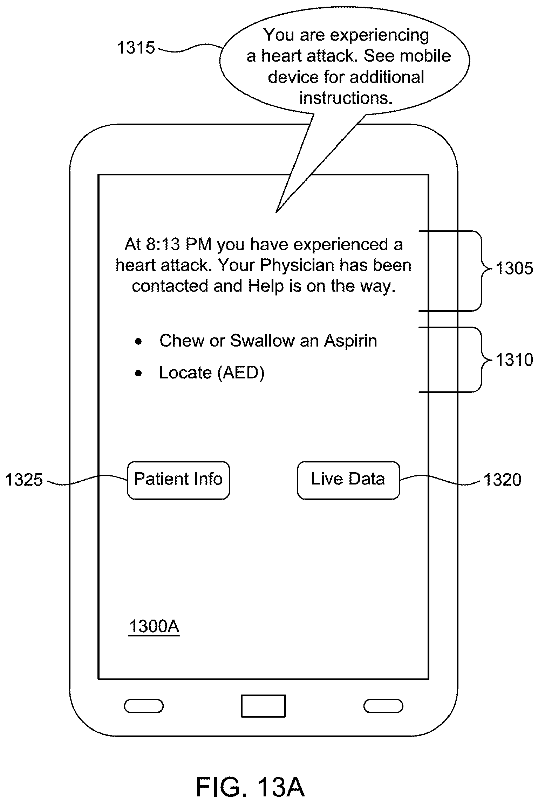

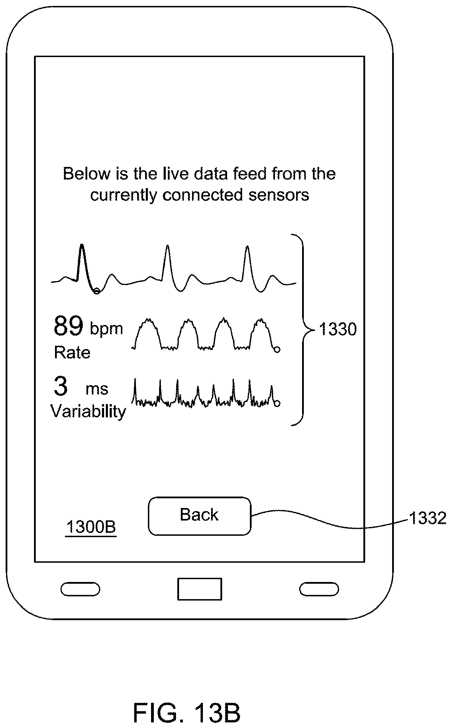

As shown at block 1070, the user 802 may be alerted that a critical event has occurred and may be instructed on how to respond to the event. For example, the local computing device 106 may display a message 1305 as shown in FIG. 13A. Additionally or alternatively, the local computing device 106 may play an audible message indicating that the critical event has occurred. For example, the local computing device 106 may play a message 1315 saying "you are experiencing a heart attack. See local computing device for additional instructions." The audible message 1315 may be used to not only alert the user 802 of the critical condition but may also alert the first responders that additional information is available about the critical event. For example, FIG. 13B shows additional information 1330 about the user 802 that may be displayed on the local computing device 106 to assist the first responders to the existing medical conditions of the 802. In addition, the first responders may be assisted by the local computing device 106 displaying the data from continuous real-time data from the monitoring and processing apparatus 102 and the additional sensing apparatuses 804.

When a critical event is detected (e.g., at block 1045 of method 1000), display 1300A may be displayed on the local computing device 106 as shown in FIG 13A. The display 1300A shows example information that may be displayed if the user 802 is experiencing a heart attack. For instance, text area 1305 may display text describing the event. The display 1300A further includes actions 1310 the user 802 should take while waiting for the emergency services provider. In addition, in some instances, the local computing device 106 may provide audible instructions 1315 to the user 802. When the live selector 1320 is selected, the local computing device 106 may display the live data display 1300B. Selecting the patient history selector 1325 may cause the local computing device 106 to display the patient information display 1300C.

FIG. 13B shows an example of the live data display 1300B that may be shown on the local computing device 106 in response to the user input via selector 1320. The live data display 1300B may include the data measured by the monitoring and processing apparatus 102 and the additional external sensing apparatuses 804 in a live data portion 1330. The data may be displayed in the form of a graph or numerically. The live data portion 1330 update in real-time as the monitoring and processing apparatus 102 and the additional external sensing apparatuses 804 are set to continuously transmit in block 1065 of method 1100. Selecting the back selector 1332 causes the local computing device 106 to return to display 1300A.

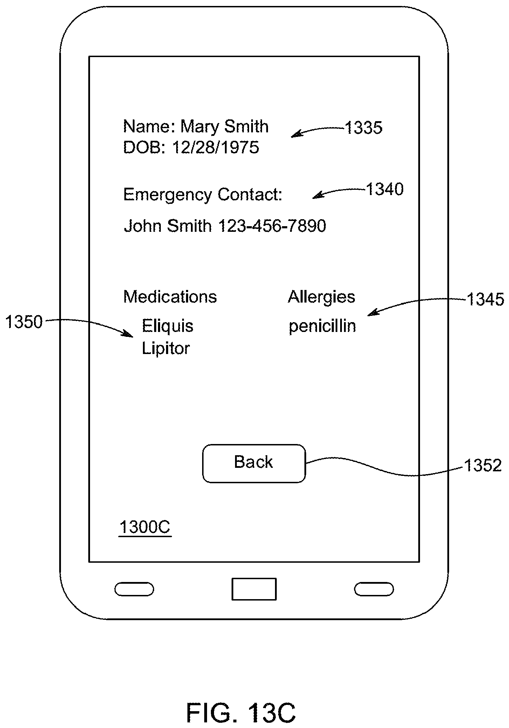

The patient information display 1300C is illustrated in FIG. 13C. The patient information display 1300C may include basic patient information 1335 and emergency contact information 1340. The patient information display 1300C may also include a list of current medications 1345 and allergies 1350 of the user 802. The patient information 1335, emergency contact information 1340, current medications 1345, and allergies 1350 may be downloaded by the local computing device 106 from the database 9100 of the cloud database 812. Selecting the back selector 1352 may cause the local computing device 106 to return to display 1300A.

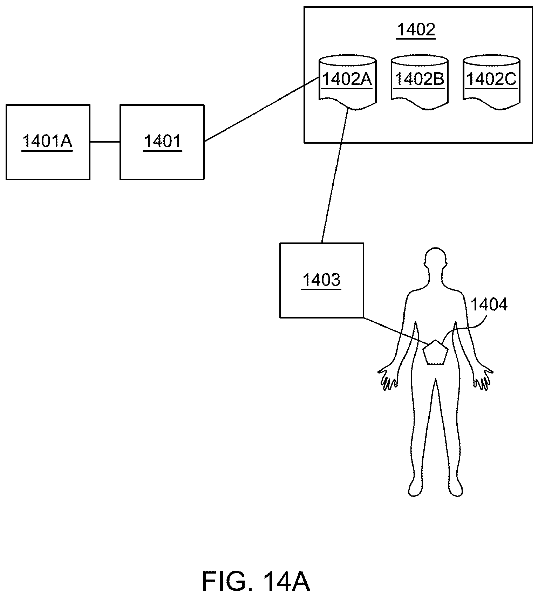

According to an embodiment, a healthcare professional dashboard 1401, of FIG. 14A, may be accessible to one or more healthcare professionals at a remote site (e.g., a doctor's office, a hospital, a medical facility, or a residential dwelling). The healthcare professional dashboard 1401 may provide constantly or periodically updated patient information via a display 1401A or to a display. The information provided via the healthcare professional dashboard 1401 may be provided via database 1402, which may include multiple databases such as 1402A, 1402B, and 1403B, which may be the same as or similar to database 9100 of FIG. 9. The database 1402 may receive and analyze the information provided from one or more local computing devices 1403 associated with one or more monitoring and processing apparatus 1404.

The information provided via a healthcare professional dashboard 1401 may be provided in compliance with any applicable regulations including General Data Protection Regulation (GDPR) or Health Insurance Portability and Accountability Act (HIPAA requirements) regulations. A healthcare professional dashboard 1401 may be configured based on access settings such that only healthcare professionals who are authorized to see a specific patient's information (e.g., patient biometric data, patent event detection data, patient history, or patient bibliographical data,) are provided access to the patient specific data via the healthcare professional dashboard 1401. According to an embodiment, all or a part of the data provided via a healthcare professional dashboard is anonymized prior to being provided via the healthcare professional dashboard. Such anonymized data may be provided by one or more anonymized data tables 915 from one or more databases 9100 of FIG. 9.

According to an embodiment, a healthcare professional dashboard 1401, of FIG. 14A, may be configured to receive input (e.g., via a touch screen, mouse, keyboard, or voice activated device). The input may be provided by a health care professional and may cause an instruction to be sent to local computing device 1403 via a network such as network 120 of FIG. 1. The instruction may then be provided to monitoring and processing apparatus 1404 via a network such as network 110 of FIG. 1. The instruction may cause the monitoring and processing apparatus 1404 to take an action such as switch from a first, general, operation mode to a second, continuous, operation mode such that the monitoring and processing apparatus 1404 may transmit patient biometric data to local computing device 1403 on a constant basis instead of on a periodic basis. The health care professional may provide the input based on, for example, one or more concerning data points that the health care professional sees on the healthcare professional dashboard 1401.

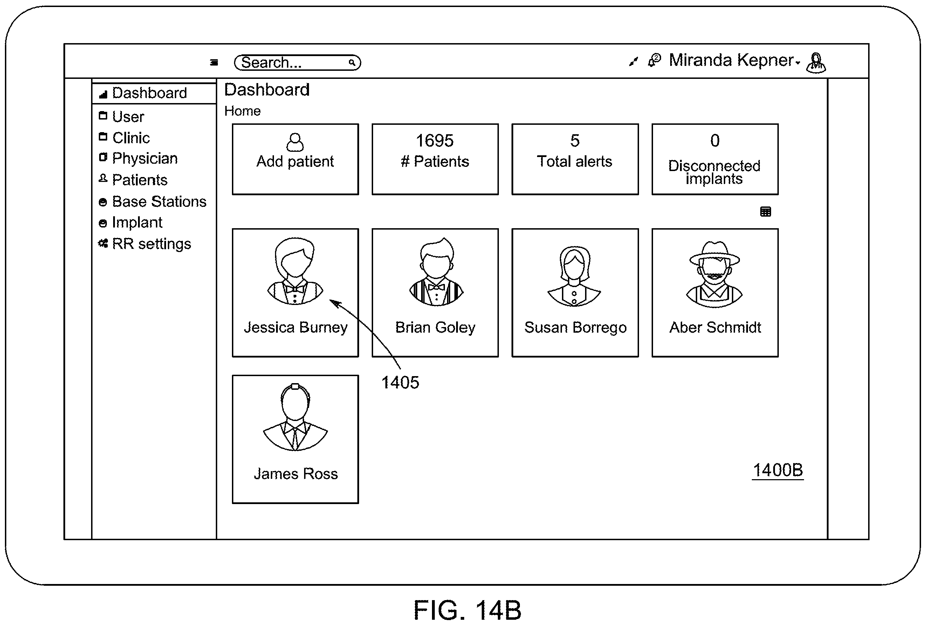

FIG. 14B shows an example doctor dashboard 1400B that is accessible by the doctor logging into the doctor portal 808 of FIG. 8. In some instances, the doctor can access the dashboard 1400B from a standard computer browser. In other instances, the dashboard 1400B is only accessible via a proprietary application running on either a computer or a local computing device. The dashboard 1400B allows the doctor to view information stored in database 1402 about a particular patient. For example, if the doctor selects "Jessica Burney"1405, the patient details page 1400C is displayed.

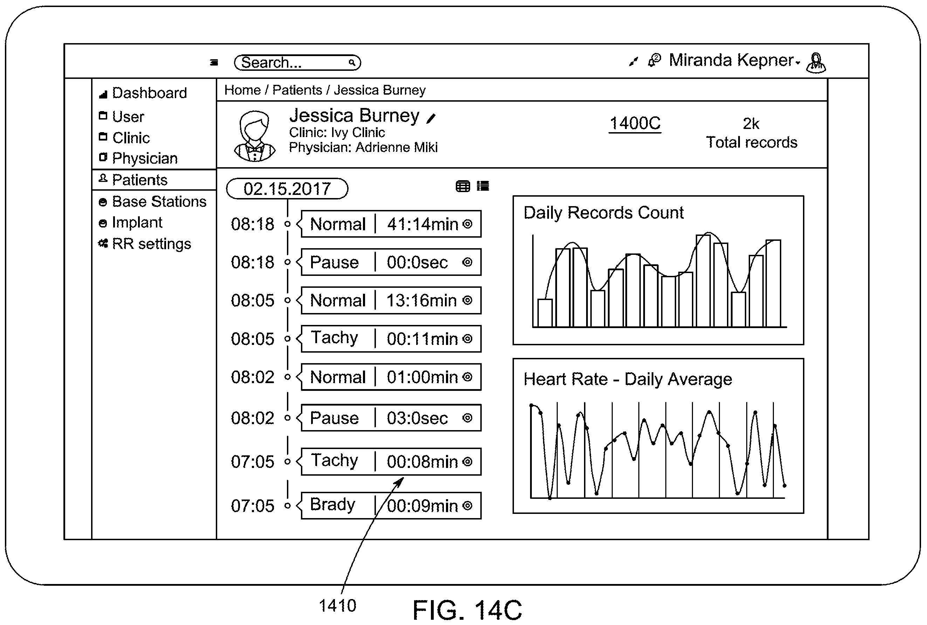

FIG. 14C illustrates an example of patient details page 1400C. The patient details page 1400C displays a graphical summary of the sensor data retrieved from a table in database 1402 that is associated with a particular user. For example, 1400C shows the sensor data for "Jessica Burney." The patient details page 1400C further includes a summary of events that the patient has experienced. Clicking on of the events 1410 causes the event details page 1400D to be displayed.

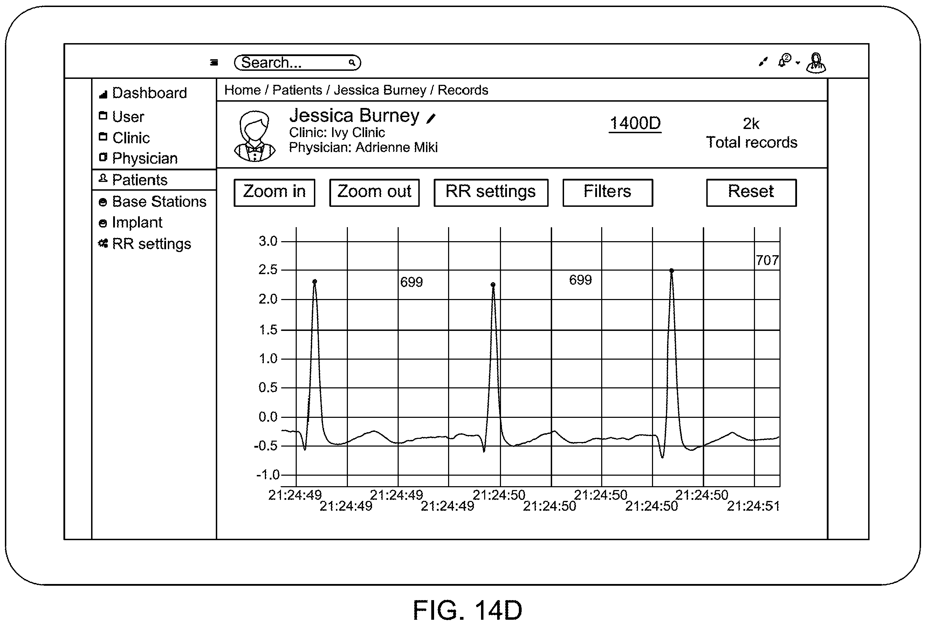

An example of the event details page 1400D is illustrated in FIG. 14D. The event details page 1400D provides a graphic summary of the data that is stored in the database 1402 that is associated with a particular event.

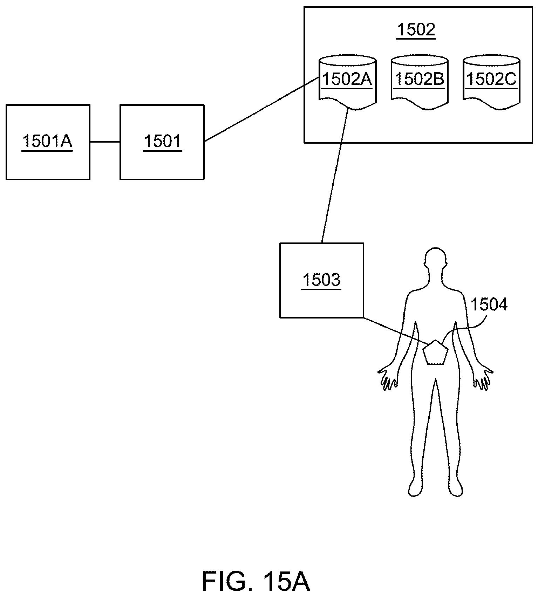

According to an embodiment, a patient dashboard 1501, such as shown in FIG. 15A, may be accessible to one or more patients. The patient dashboard 1501 may provide constantly or periodically updated patient information via a display 1501A or to a display. The information provided via the patient dashboard 1501 may be provided via database 1502, which may include multiple databases such as 1502A, 1502B, and 1503B, which may be the same as or similar to database 9100 of FIG. 9. It should be noted that database 1502 may be the same as database 1402 of FIG. 14A, which provides data to healthcare professional dashboard 1401. The database 1502 may receive and analyze the information provided from one or more local computing devices 1503 associated with one or more monitoring and processing apparatus 1504.

The information provided via patient dashboard 1501 may be provided in compliance with any applicable regulations including General Data Protection Regulation (GDPR) or Health Insurance Portability and Accountability Act (HIPAA requirements) regulations. A patient dashboard 1501 may be configured based on access settings such that only individuals (e.g., patient or family members) who are authorized to see a specific patient's information (e.g., patient biometric data, patent event detection data, patient history, or patient bibliographical data) may be provided access to the patient specific data via the patient dashboard 1501.

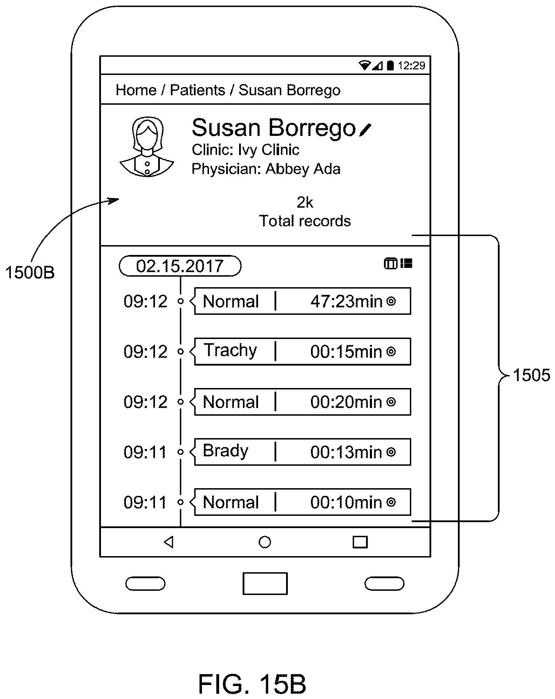

FIG. 15B illustrates a user interface 1500B that may be accessible to the user 802, of FIG. 8, via the patient portal 806. The user 802 may acces the use interface 1500B by logging into the patient portal 806. In some instances, the patient portal is accessible via a standard browser. In other instances, access to the patient portal 806 is restricted only to a proprietary application running on either a computer or a local computing device. The user interface 1500A may allow the user to retrieve and view their information stored in the database 1502 such as the sensor data stored in a table within database 1502. For example, the user interface 1500B may include several selectable periods 1505 of data acquired by the sensors 212, of FIG. 2, stored in the database 9100.

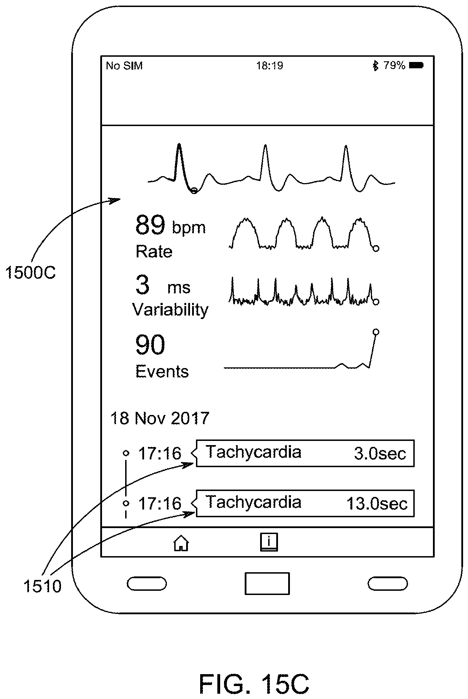

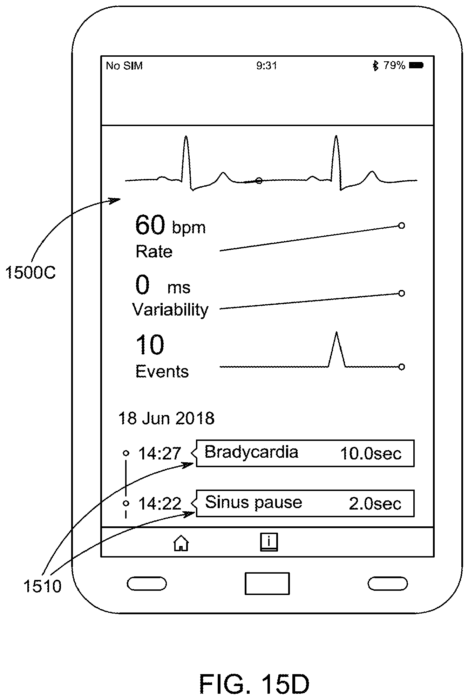

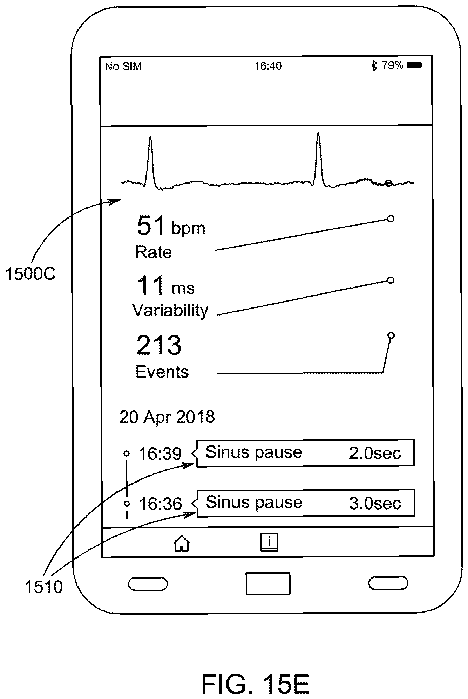

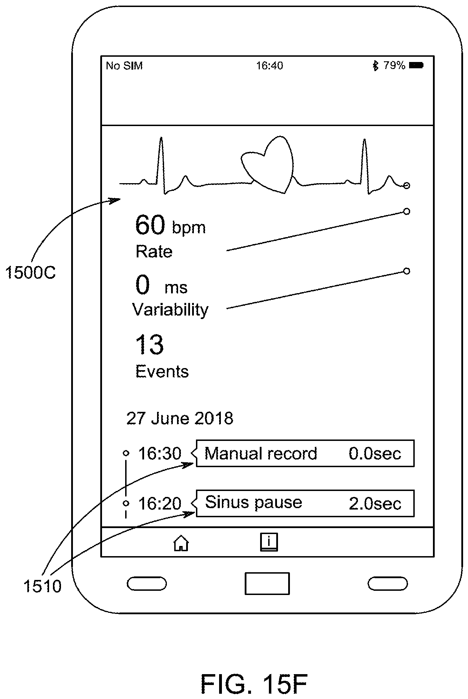

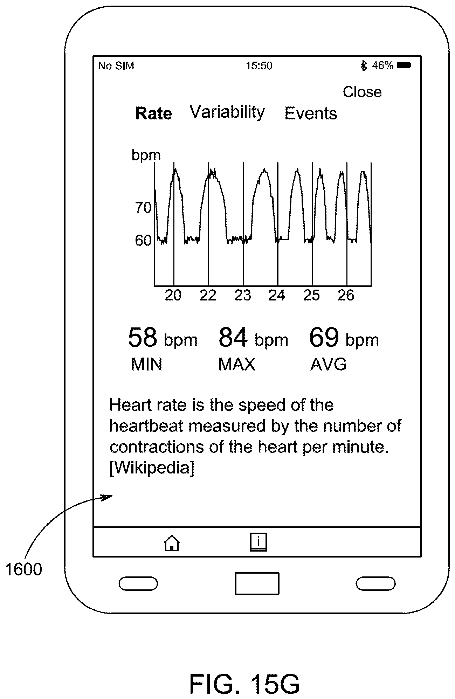

FIGs. 15C, 15D, 15E and 15F illustrate different examples of a record detail page 1500C. If the user 802 selects a particular recording period (e.g., one of the selectable periods 1505 shown in FIG. 15B), a record detail page 1500C may be displayed. For example, FIG. 15C shows a particular record detail page 1500C for a recording period during which the user 802 experienced tachycardia at 17:16. If the user clicks on the tachycardia event 1510, the details of the event may be displayed (e.g., displayed on an event detail page 1600, an example of which is shown in FIG. 15G). In addition, the record detail page 1500C may show a history graph of the number of the events per day in the last week/month during the particular recording period.

Similarly, in the example record detail page 1500C shown in FIG. 15D, the user 802 experienced a bradycardia event at 14:27 and a sinus pause event at 14:22. If the user selects either of these events 1510, the details of the event may be displayed (e.g., on an event detail page 1600). Likewise, in the example record detail page 1500C shown at FIG. 15E, the user 802 experienced a sinus pause event at 16:39 and 16:36. If the user selects either of these events 1510, the details of either event may be displayed (e.g., on an event detail page 1600). The example record detail page 1500C shown in FIG. 15F indicates that the user 802 experienced a manual record event at 16:30 and a sinus pause event at 16:20. If the user selects either of these events 1510 displayed on the record detail page 1500C, the details of either event 1510 may be displayed (e.g., on an event detail page 1600).

FIG. 15G shows an example event detail page 1600. The event detail page 1600 may provide the data recorded by the sensors 212 when a particular event occurred. In some examples, the event detail page 1600 includes clinical information about the particular event experienced by the user. The clinical information may be, for example, retrieved from the database 9100 or be retrieved from public data sources such as Wikipedia or WebMD via an API integration.

Any of the functions and methods described herein can be implemented in a general purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. Although features and elements are described above in particular combinations, each feature or element can be used alone without the other features and elements or in various combinations with or without other features and elements.

## Claims

1. A system for biometric monitoring and processing, the system comprising:
a monitoring and processing apparatus comprising a memory, a sensor and a processor, the processor configured to:
receive biometric patient data from the sensor, and
transmit, via a first network, the biometric patient data based on a triggering cause;
a local computing device configured to receive the biometric patient data via the first network, generate a combined patient data based at least on the received biometric patient data, and transmit the combined patient data via a second network; and
a remote computing system configured to:
receive the combined patient data via the second network,
determine that a critical event is detected based on the combined patient data, and
initiate a critical event action based on determining that the critical event is detected.

2. The system of claim 1, wherein the monitoring and processing apparatus is one of an external device or an implantable device.

3. The system of claim 1, wherein the memory is configured to store the biometric data for at least one of a pre-determined time or a dynamically determined time.

4. The system of claim 1, further comprising an additional sensing apparatus configured to:
receive a request for an additional data from the local computing device;
sense the additional data; and
transmit, via the first network, the additional data.

5. The system of claim 4, wherein the combined data is further based on the additional data.

6. The system of claim 1, wherein the monitoring and processing apparatus is configured to be in one of a general operation mode or a continuous operation mode.

7. The system of claim 6, wherein the monitoring and processing apparatus further comprises a transceiver configured to transmit biometric patient data at a first frequency based on the general operation mode or a second frequency based on the continuous operation mode.

8. The system of claim 1, wherein initiating a critical event action comprises transmitting, via the second network, a critical event action instruction comprising one of contacting a healthcare professional, setting the monitoring and processing apparatus to a continuous operation mode, or providing instructions to a user.

9. The system of claim 8, further comprising:
receiving, via the second network and at the local computing device, the critical event action instruction; and
performing an action based on the critical event action instruction.

10. A monitoring and processing apparatus, the apparatus comprising:
a patch comprising:
an adhesive component;
an electronic device comprising electronic circuitry;
a first portion configured to receive a removable electronic device; and
a second portion coupled to the first portion and separated from the first portion by a gap, the second portion comprising an electrode extending from the first portion to the second portion across the gap,
the electronic device electronically coupled to the attachable patch via the at least one electrode.

11. The apparatus of claim 10, wherein the electronic device is electrically connected to the electrode by at least one conductive via disposed between the electronic circuitry and the electrode.

12. The apparatus of claim 10, wherein the electronic device is configured to receive at least one of a user input and a biometric patient data.

13. The apparatus of claim 12, wherein the electronic device further comprises a transceiver configured to transmit the biometric patient data.

14. The apparatus of claim 13, wherein the transceiver is configured to transmit the biometric patient data based on the user input.

15. A method comprising:
receiving, at a local computing device and via a first network, biometric patient data sensed by a sensor, based on a triggering cause;
generating, at the local computing device, a combined patient data based at least on the received biometric patient data;
transmitting the combined patient data via a second network; and
receiving a critical event action from a remote computing device, at the local computing device and via the second network, the critical event action determined based on the combined patient data.

16. The method of claim 15, wherein the first network is a local area network and the second network is a wide area network.

17. The system of claim 1 or the method of claim 15, wherein the combined patient data is further based on at least one of an additional data or the triggering cause.

18. The method of claim 15, wherein the sensor is part of a monitoring and processing apparatus.

19. The method of claim 15, wherein the critical event action comprises one of contacting a healthcare professional, setting a monitoring and processing apparatus to a continuous operation mode, or providing instructions to a user.
